# EUROPÄISCHE PATENTSCHRIFT

(11) **EP 1 893 755 B1**
(45) Veröffentlichungstag und Bekanntmachung des Hinweises auf die Patenterteilung: **14.01.2009**
(21) Anmeldenummer: 06754335.5
(22) Anmeldetag: 13.06.2006
(51) Int. Cl.: C12N 9/52

(54) **VERFAHREN ZUR HERSTELLUNG VON LIPASEN**
LIPASE PRODUCTION METHOD
PROCEDE POUR PRODUIRE DES LIPASES

(30) Priorität: 15.06.2005 DE 102005027685
(43) Veröffentlichungstag der Anmeldung: 05.03.2008
(73) Patentinhaber: BASF SE, 67056 Ludwigshafen (DE)
(72) Erfinder: BESELIN, Anke, 45481 Mülheim (DE); ROSENAU, Frank, 40880 Ratingen (DE); JÄGER, Karl-Erich, 45479 Mülheim an der Ruhr (DE); BREUER, Michael, 64285 Darmstadt (DE); HAUER, Bernhard, 67136 Fussgönheim (DE)
(74) Vertreter: Reitstötter - Kinzebach
(86) Internationale Anmeldenummer: PCT/EP2006/005668
(87) Internationale Veröffentlichungsnummer: WO 2006/133900

(56) Entgegenhaltungen:
- DATABASE UniProt 1. März 2002 (2002-03-01), PERNA NT ET AL.: "Hypothetical protein yhbO" XP002401767 Database accession no. Q8XA99_ECO57 in der Anmeldung erwähnt & PERNA N T ET AL: "GENOME SEQUENCE OF ENTEROHAEMORRHAGIC ESCHERICHIA COLI O157:H7" NATURE, NATURE PUBLISHING GROUP, LONDON, GB, Bd. 409, Januar 2001 (2001-01), Seiten 529-533, XP002943756 ISSN: 0028-0836
- EL KHATTABI M ET AL: "Role of the lipase-specific foldase of Burkholderia glumae as a steric chaperone." THE JOURNAL OF BIOLOGICAL CHEMISTRY. 1 SEP 2000, Bd. 275, Nr. 35, 1. September 2000 (2000-09-01), Seiten 26885-26891, XP002401765 ISSN: 0021-9258

## Beschreibung

Die Erfindung betrifft neuartige Proteine, insbesondere mit Proteaseaktivität, welche die Produktion extrazellulärer Lipasen durch Bakterien, insbesondere der Gattung *Burkholderia* fördern, dafür kodierende Nukleinsäuresequenzen, diese enthaltende Expressionskonstrukte, damit transformierte Wirte, Verfahren zur Herstellung der Proteine mit Proteaseaktivität sowie Verfahren zur Herstellung von Lipasen, insbesondere durch Bakterien der Gattung *Burkholderia.*

### Hintergrund der Erfindung

Proteasen sind in der Natur weit verbreitet und weisen eine große Vielzahl physiologischer Funktionen auf. Es handelt sich hierbei um abbauende Enzyme, welche die Spaltung von Peptidbindungen katalysieren. Während extrazelluläre Proteasen große Proteine in kleinere Moleküle für eine anschließende Absorption spalten, spielen intrazelluläre Proteasen eine wichtige Rolle bei der Stoffwechselregulation. Deren proteolytische Aktivität umfasst beispielsweise die Aktivierung zymogener Formen von Enzymen durch limitierte Proteolyse, die Prozessierung und den Transport von sekretorischen Proteinen über die Membranen hindurch oder die Regulation der Genexpression durch Abbau regulatorischer Proteine oder Modifikation ribosomaler Proteine [16].

Im Jahre 2000 wurden verschiedene Proteasen im Genom von *Pseudomonas aeruginosa* identifiziert, welche eine signifikante Homologie mit den Proteasen DegP, Prc, Protease III und SohB von *Escherichia coli* zeigten. Nach Inaktivierung der entsprechenden Gene im Genom von *P. aeruginosa* zeigten drei Mutanten eine höhere Lipaseaktivität, während eine Mutante eine niedrigere Lipaseaktivität im Überstand aufwies. Weitere Experimente ergaben, dass die Genexpression des LipAB-Operons nicht beeinflusst wurde, was bestätigte, dass diese Proteasen die Faltung und/oder Sekretion der Lipase LipA in *P. aeruginosa* beeinflussen [24].

*Burkholderia glumae* ist ein Pflanzenpathogen, welcher in Reispflanzen Hülsenfäule und Mehltau an den Keimlingen und Rispen verursacht. Wie viele andere Bakterien produziert *B. glumae* eine extrazelluläre Lipase (EC 3.1.1.3), welche sich als nützlich für eine Vielzahl verschiedener biotechnologischer Applikationen erwies [18].

Die Produktion dieser Lipase mit hoher Ausbeute wäre deshalb wünschenswert. Um einen geeigneten überexprimierenden Stamm zu konstruieren ist es erforderlich, potenzielle Engstellen der Lipaseproduktion zu identifizieren. Diese Engstellen können auf der Ebene der Genexpression, der Faltung und der Sekretion des Enzyms in das Kulturmedium erwartet werden.

Es war deshalb Aufgabe der Erfindung, einen Weg zur Verbesserung der extrazellulären Lipaseproduktion durch *B. glumae* zu finden.

### Kurzfassung der Erfindung

Obige Aufgabe wurde durch Bereitstellung eines Proteins gemäß den Patentansprüchen gelöst, das einen positiven Effekt auf die Expression und/oder Faltung und Sekretion der Lipase lipA in *B. glumae* bewirkt.

Erfindungsgemäß wurde zunächst eine Cosmid-Bibliothek von *B. glumae* PG1 unter Verwendung des Vektors pLAFR3 [20] mit breiter Wirtsspezifität konstruiert. Nach Screening von etwa 2500 Klonen wurden 15 Cosmide identifiziert, welche die Lipaseproduktion beeinflussen. Die entsprechenden DNA-Fragmente von 2 Cosmiden wurden in einen Vektor mit breiter Wirtsspezifität subkloniert, wobei man 5 verschiedene Plasmide erhielt. Die Expression dieser Plasmide in *B. glumae* ergab ein Klon, der eine erhöhte Lipaseaktivität zeigte. Nach DNA-Sequenzierung des entsprechenden DNA-Fragments wurden die offenen Leserahmen (orfs) unter Verwendung des Open-Reading-Frame-Suchprogramms (ORF Finder) des National Center of Biotechnology Information (NCBI) identifiziert. Der längste ORF umfasste 540 Basenpaare und die davon abgeleitete Aminosäuresequenz enthielt eine konservierte Domäne, welche man in der ThiJ/Pfpl-GFamilie findet. Diese Familie umfasst verschiedene Proteine, wie Proteasen, Transkriptionsregulatoren, RNA-Bindungsproteine oder Chaperone [3]. Es wird deshalb angenommen, dass dieser ORF für ein bisher noch nicht charakterisiertes Protein kodiert, welches eine zytoplasmatische Protease darstellt, die einen positiven Effekt auf die Expression und/oder Faltung und Sekretion der Lipase lipA in *B. glumae* bewirkt.

### Figurenbeschreibung

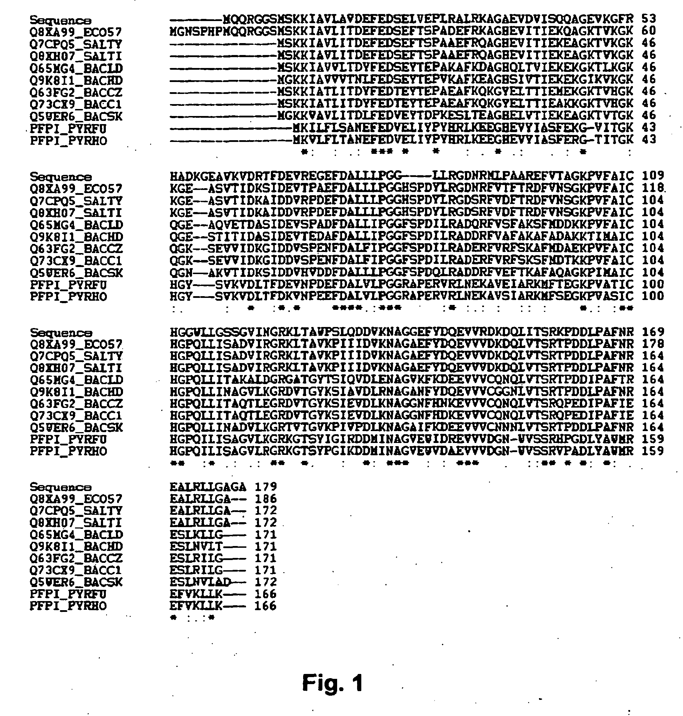

In den Figuren zeigt
- Figur 1: ein mehrfaches Sequenzalignment eines ORF, der für eine Protease aus *Burkholderia glumae* kodiert mit Proteinen aus Datenbanken. Zunächst wurde die Aminosäuresequenz der Protease einer Sequenz-Ähnlichkeitsrecherche unter Verwendung des WU-BLAST2-Programms von EBL-EMBI mit einer BLOSUM62-Matrix unterzogen. Die Proteine mit hoher Ähnlichkeit zur Protease wurden anschießend zur Erstellung eines multiplen Sequenzalignments unter Verwendung von Db-Clustal von EBL-EMBI ausgewählt. Reste innerhalb einer Spalte, die in allen Sequenzen des Alignments identisch sind, wurden mit einem Stern markiert. Ein Doppelpunkt steht für eine konservative Substitution. Semi-konservative Substitutionen sind durch einen einzelnen Punkt gekennzeichnet. In der ersten Zeile ist die erfindungsgemäße Protease dargestellt. In den folgenden Zeilen sind angegeben: Q8XA99: Hypothetisches Protein yhbO aus *Escherichia coli.* Q7CPQ5: Putative intrazelluläre Proteinase XHBO aus *Salmonella typhimurium.* Q8XH07: Hypothetisches Protein STY3452 aus *S. typhimurium.* Q5WER6: Allgemeines Stressprotein GSP18 aus *Bacillus clausii.* Q65MG4: YfkM aus *B. lichenformis.* Q9K811: Allgemeines Stressprotein aus B. halodurans BH3025. Q370CX9: ThiJ/Pfpl-Familien-Protein aus *B. cereus* (Stamm ATCC 10987) BCE0935. Q65FG2: Protease I, ThiJ/Pfpl-Familien-Protein aus *B. cereus.* PFI_PYRHO: Protease I aus *Pyrococcus horikoshii* OT3. PFPI _PYRFU: Protease I aus *Pyrococcus furiosus.*

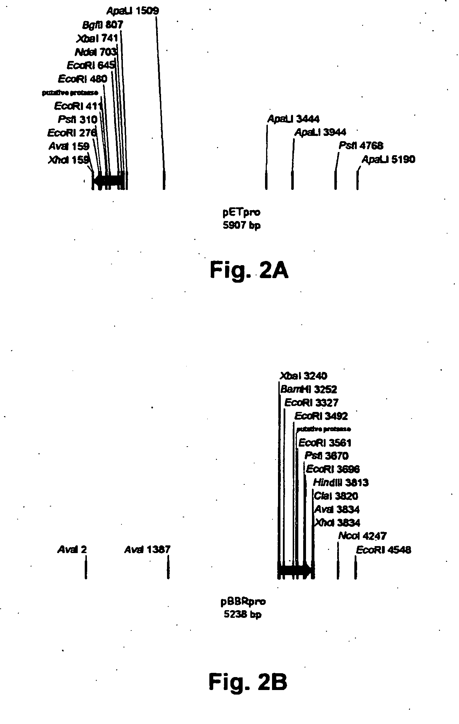

- Figur 2: die Plasmidkarten erfindungsgemäß verwendeter Plasmidkonstrukte, und zwar: Figur 2A das Plasmid pITpro und Figur 2B dasPlasmid pBBRpro.

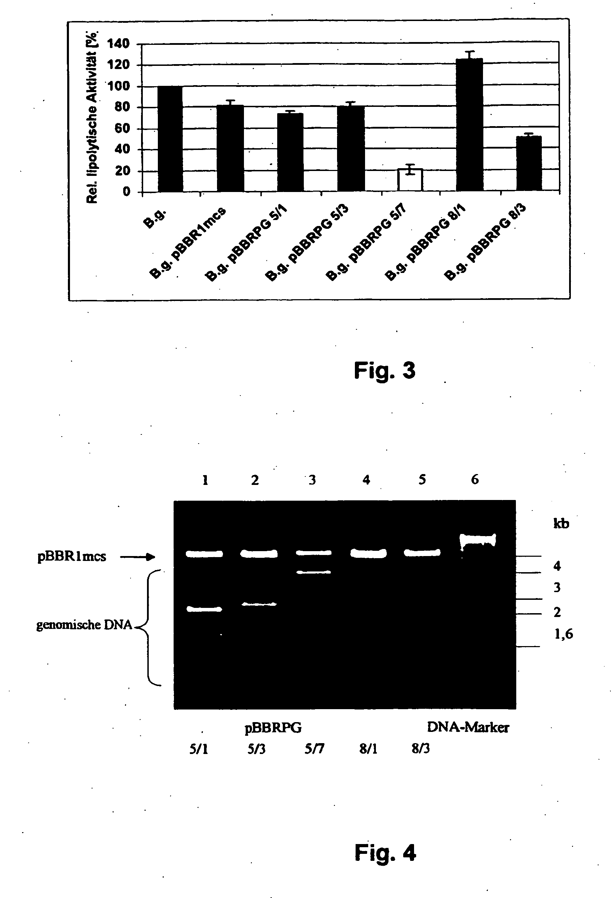

- Figur 3: die relative lipolytische Aktivität, beobachtet bei Expression verschiedener Plasmide, welche subklonierte DNA aus *B. glumae* PG1 enthielten. Die Fehlerbalken zeigen die Standardabweichung aus 4 getrennten Experimenten. Die Lipaseaktivität wurde spektrophotometrisch mit p-Nitrophenylpalmitat als Substrat bestimmt und ist als relative lipolytische Aktivität relativ zum Wildtypstamm gezeigt [21]. Als Kontrolle diente der leere ExpressionsvektorpBBR1mcs. Die Expression des Plasmids pBBRPG8/1, welches das Gen für die erfindungsgemäße Protease enthielt, führte zu einer Zunahme der Lipaseaktivität um 20 bis 30 %.
- Figur 4: die Restriktionsanalyse der Plasmide pBBRPPG 5/1, 3,7,8/1 und 3 unter Verwerdung der Restriktionsenzyme XbaI und HincII. 1 µg DNA wurde isoliert und auf ein 0,8 %-iges Agarose-Gel aufgetragen. Die DNA-Marker wurden von Invitrogen bezogen.

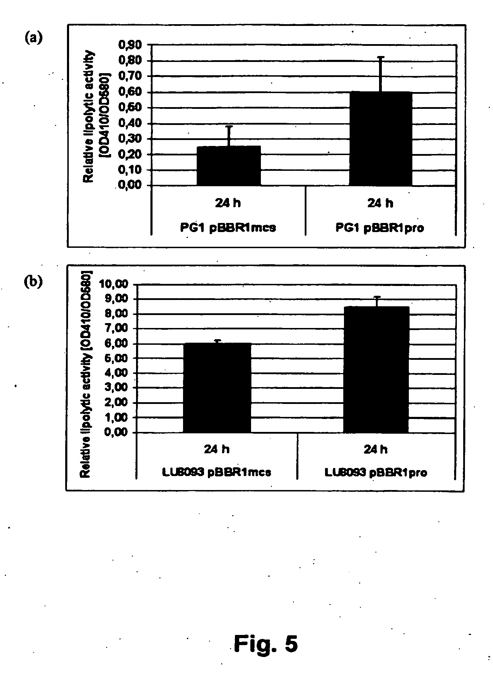

- Figur 5: die Homologe Expression der putativen Protease in *B. glumae* PG1 (a) und LU8093 (b). Die Expression erfolgte in vier separaten Experimenten unter der Verwendung von PG-Medium mit 1% Olivenöl als Induktor der Lipaseproduktion. Nach 24 Stunden wurden die Kulturen geerntet und die OD₅₈₀ bestimmt. Die Bestimmung der extrazellulären Lipaseaktivität erfolgte spektrophotometrisch unter der Verwendung von p-Nitrophenylpalmitat als Substrat (nach Winkler *et al.,* 1978 [31]). Die relativen lipolytischen Aktivitäten wurden durch Korrelation von Δ410/min mit der OD₅₈₀ berechnet. Zur Kontrolle wurden die Stämme ebenfalls mit dem Leervektor pBBR1mcs transformiert und mitgeführt.

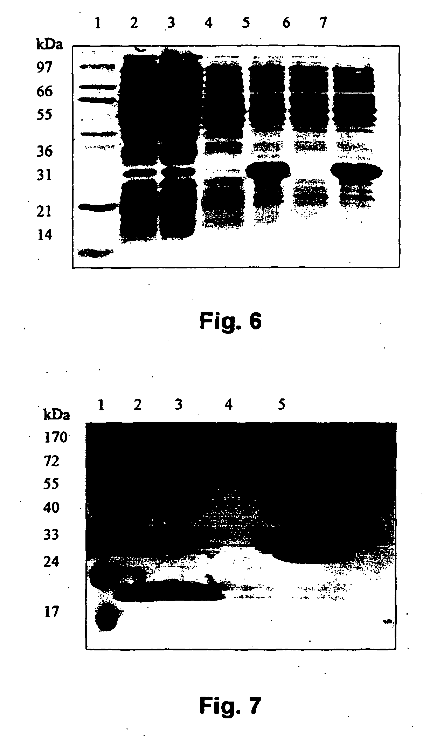

- Figur 6: die Überexpression der erfindungsgemäßen Protease in *E. coli* BL12DE3. Proben wurden nach 0, 2 und 4 Stunden (T₀, T₂, T₄) genommen und durch 15 %-ige SDS-PAGE aufgetrennt. Das Gel wurde mit Coomassie Blue R-250 angefärbt. Bahn 1: Proteinstandard M12 (Invitrogen), 2: T₀ BL21DE3 pET22b, 3: T₀ BL21DE3 pETpro, 4: T₂ BL21DE3 pET22b, 5: T₀ BL21 DE3 pETpro, 6 : T₄ BL21DE3 pET22b, 7 : T₄ BL21 DE3 pETpro.
- Figur 7: die SDS-PAGE-Analyse für jeden Reinigungsschritt. Bahn 1: Proteinstandard PageRuler (Fermentas) 2: 10µl Zelllysat (1:5). 3: 10 µl Durchlauf. 4: 10 µl Waschfraktion. 5: 10 µl Eluat (1:13).

### Detaillierte Beschreibung der Erfindung:

1. Bevorzugte Ausführungsformen
   Ein erster Gegenstand der Erfindung betrifft Proteine mit dem Merkmalen gemäß Hauptanspruch welche wenigstens eine der folgenden zusätzlichen Eigenschaften aufweisen können:
   a) eine Aminosäuresequenz, umfassend die Consensus-Sequenz: AICHGP (SEQ ID NO: 7)
   b) keine Helix-Turn-Helix (HTH) DNA Bindungsdomäne;
   c) ein Molekulargewicht von etwa 20-22 kDa, bestimmt durch SDS-PAGE unter denaturierenden Bedingungen.
   Beispielsweise können ein oder zwei oder drei der Merkmale a),b) und c) in beliebiger Kombination vorliegen, insbesondere können diese Merkmale gleichzeitig ausgebildet sein.
   Weiterhin kann das erfindungsgemäße Protein Proteaseaktivität umfassen.

Vorzugsweise besitzen die erfindungsgemäßen Proteine einen pl-Wert im Bereich von 5,4 bis 5,5. Sie sind zugänglich aus Bakterien der Gattung *Burkholderia,* insbesondere *Burkholderia glumae.*

Die erfindungsgemäßen Proteine sind dadurch gekennzeichnet, dass sie nach Expression in einem extrazelluläre Lipase produzierenden bakteriellen Wirt, wie Bakterien der Gattung *Burkholderia,* insbesondere in *Burkholderia glumae,* die extrazelluläre lipolytische Aktivität, bestimmt unter Standardbedingungen, erhöhen. Dabei wird die extrazelluläre lipolytische Aktivität um wenigstens etwa 1%, wie z. B. etwa 5 bis 200% oder 10 bis 100 % oder 20 bis 50 %, im Vergleich zum Grundwert erhöht wird.

Gegenstand der Erfindung sind auch Proteine, kodiert von einer Nukleinsäure, umfassend SEQ ID NO: 1 oder eine davon abgeleitete Sequenz mit wenigstens 80% Sequenzhomologie, sowie diese kodierenden Nukleinsäuresequenzen selbst.

Ein weiterer Gegenstand der Erfindung betrifft Expressionsvektoren, umfassend unter der genetischen Kontrolle wenigstens einer regulativen Nukleinsäuresequenz eine ein Protein mit Proteaseaktivität kodierende Nukleinsäuresequenz gemäß obiger Definition.

Ein weiterer Gegenstand der Erfindung ist ein rekombinanter Mikroorganismus, genetisch modifiziert mit wenigstens einem Expressionsvektor nach obiger Definiton.

Die Erfindung betrifft weiterhin ein Verfahren zur Herstellung eines Proteins mit Proteaseaktivität nach obiger Definiton, wobei man einen rekombinanten Mikroorganismus unter dieses Protein exprimierenden Bedingungen kultiviert und das gebildete Protein isoliert.

Gegenstand der Erfindung ist außerdem ein Verfahren zur Herstellung einer vorzugsweise extrazellulären Lipase (E.C. 3.1.1.3), wobei man einen zur Produktion dieser Lipase befähigten Wirt zur Expression eines funktionalen Proteins mit Proteaseaktivität nach obiger Defintion veranlasst und gleichzeitig oder zeitlich versetzt zur Lipase-Expression veranlasst und die gebildete Lipase isoliert. Insbesondere ist dabei der Wirt ein Bakterium der Gattung *Burkholderia,* insbesondere *Burkholderia glumae.*

Die dabei hergestellte Lipase umfasst gemäß einer bevorzugten Variante eine Aminosäuresequenz gemäß SEQ ID NO: 6 oder eine davon abgeleitete Aminosäuresequenz mit wenigstens 80% Sequenzhomologie, oder wird von einer Nukleinsäuresequenz, umfassend eine Sequenz gemäß SEQ ID NO: 5 oder eine davon abgeleitete Nukleinsäuresequenz mit wenigstens 80% Sequenzhomologie, kodiert.

### 2. Erläuterung allgemeiner Begriffe

Ein "Protein mit Proteaseaktivität" zeigt in wenigstens einem der hierin beschriebenen Testverfahren die enzymatische Aktivität einer Protease (proteolytische Aktivität), wie z.B., ohne darauf beschränkt zu sein, eine proteolytische Aktivität bestimmt mit wenigstens einem geeigneten Protease-Substrat. Als nichtlimitierende Beispiele können genannt werden Aminopeptidase-Substrate, wie Lysin-β-Na, Arginin-β-Na, L-Alanin-β-Na, Glutamat(βNa)-OH. Die enzymatische Aktivität des Proteins muss aber nicht auf die proteolytische Aktivität beschränkt sein.

Unter einer "HTH Bindungsdomäne" versteht man ein Helix-turn-helix DNA-Bindungsmotif in Proteinsequenzen, wie beschrieben von Dodd et al. (1990) in [26], worauf hierin ausdrücklich Bezug genommen wird.

"Extrazelluläre Lipasen" in Sinne der Erfindung sind insbesondere solche der Enzymklasse E.C. 3.1.1.3. Bevorzugt werden diese von Bakterien der Gattung *Burkholderia,* insbesondere von *Burkholderia glumae* produziert, wie insbesondere die Lipase LipA. Derartige Lipasen finden insbesondere in biotechnologischen Prozessen Anwendung, wie z.B. beschrieben in Breuer et al (2004) [27], Jaeger et al. (2002 [28] und Schmid et al (2001) [29], worauf hierin ausdrücklich Bezug genommen wird.

"Extrazelluläre lipolytische Aktivität, bestimmt unter Standardbedingungen", bedeutet die ermittelte Aktivität bei Anwendung der spektrophotometrischen Standard-Bestimmungsmethode mit p-Nitrophenylpalmitat als Substrat nach Winkler et al [31] (Substrat-Endkonzentration 0,8 mM; 37°C; in Soerensen-Phosphatpuffer), wie beschrieben im experimentellen Teil.

Unter einer "abgeleiteten" Sequenz, z.B. einer abgeleiteten Aminosäure oder Nukleinsäuresequenz, wird erfindungsgemäß, wenn keine anderen Angaben gemacht werden, eine Sequenz verstanden, die mit der Ausgangssequenz eine Identität von mindestens 80% oder mindestens 90%, insbesondere 91%, 92%, 93%, 94%, 95%, 96%, 97%, 98% und 99% aufweist.

Unter "Identität" zwischen zwei Nukleinsäuren wird die Identität der Nukleotide über die jeweils gesamte Nukleinsäurelänge verstanden, insbesondere die Identität, die durch Vergleich mit Hilfe der Vector NTI Suite 7.1 Software der Firma Informax (USA) unter Anwendung der Clustal Methode (Higgins DG, Sharp PM. Fast and sensitive multiple sequence alignments on a microcomputer. Comput Appl. Biosci. 1989 Apr;5(2):151-1) unter Einstellung folgender Parameter berechnet wird:

| Multiple alignment parameter: | |
|---|---|
| Gap opening penalty | 10 |
| Gap extension penalty | 10 |
| Gap separation penalty range | 8 |
| Gap separation penalty | off |
| % identity for alignment delay | 40 |
| Residue specific gaps | off |
| Hydrophilic residue gap | off |
| Transition weighing | 0 |

| Pairwise alignment parameter: | |
|---|---|
| FAST algorithm on | |
| K-tuple size | 1 |
| Gap penalty | 3 |
| Window size | 5 |
| Number of best diagonals | 5 |

### 3. Weitere Ausgestaltungen der Erfindung

### 3.1 Erfindungsgemäße Proteine

Die vorliegende Erfindung ist nicht auf die konkret offenbarten Proteine bzw. Enzyme mit Proteaseaktivität beschränkt, sondern erstreckt sich vielmehr auch auf funktionale Äquivalente davon.

"Funktionale Äquivalente" oder Analoga der konkret offenbarten Enzyme sind im Rahmen der vorliegenden Erfindung davon verschiedene Polypeptide, welche weiterhin die gewünschte biologische Aktivität, wie z.B. proteolytische Aktivität, besitzen.

So versteht man beispielsweise unter "funktionalen Äquivalenten" Enzyme, die im verwendeten Test auf proteolytische Aktivität mindestens 20 %, bevorzugt 50 %, besonders bevorzugt 75 %, ganz besonders bevorzugt 90 % der Aktivität eines Enzyms, umfassend eine Aminosäuresequenz gemäß SEQ ID NO:2 aufweisen. Funktionale Äquivalente sind außerdem vorzugsweise von pH 4 bis 10 stabil und besitzen vorteilhaft ein pH-Optimum im Bereich von pH 5 bis 8 sowie ein Temperaturoptimum im Bereich von 20°C bis 80°C.

Die proteolytische Aktivität mit Hilfe verschiedener bekannter Tests zur Bestimmung der proteolytischen Aktivität nachgewiesen werden. Ohne darauf beschränkt zu sein, sei ein Test unter Verwendung von Magermilch-Agarplatten genannt, welche pro Liter enthielten: 30 g Magermilchpulver, 5 g Hefeextrakt, 10 g NaCl, 10 g Trypton, 15 g Agar und 1 mM IPTG. Einzelne Kolonien der Expressionskulturen oder 5 µl eines jeden Reinigungsschrittes wurden auf Agarplatten aufgetragen und über Nacht bei 30 °C (*B. glumae*) oder 37 °C (*E. coli*) inkubiert. Die proteolytische Aktivität kann durch Bildung von Höfen um die Kolonien oder Proben herum nachgewiesen werden.

Unter "funktionalen Äquivalenten" versteht man erfindungsgemäß insbesondere auch Mutanten, welche in wenigstens einer Sequenzposition der oben genannten Aminosäuresequenzen eine andere als die konkret genannte Aminosäure aufweisen aber trotzdem eine der oben genannten biologischen Aktivitäten besitzen. "Funktionale Äquivalente" umfassen somit die durch eine oder mehrere Aminosäure-Additionen, - Substitutionen, -Deletionen und/oder -Inversionen erhältlichen Mutanten, wobei die genannten Veränderungen in jeglicher Sequenzposition auftreten können, solange sie zu einer Mutante mit dem erfindungsgemäßen Eigenschaftsprofil führen. Funktionale Äquivalenz ist insbesondere auch dann gegeben, wenn die Reaktivitätsmuster zwischen Mutante und unverändertem Polypeptid qualitativ übereinstimmen, d.h. beispielsweise gleiche Substrate mit unterschiedlicher Geschwindigkeit umgesetzt werden. Beispiele für geeignete Aminosäuresubstitutionen sind in folgender Tabelle zusammengefasst:

| **Ursprünglicher Rest** | **Beispiele der Substitution** |
|---|---|
| Ala | Ser |
| Arg | Lys |
| Asn | Gln; His |
| Asp | Glu |
| Cys | Ser |
| Gln | Asn |
| Glu | Asp |
| Gly | Pro |
| His | Asn ; Gln |
| Ile | Leu; Val |
| Leu | Ile; Val |
| Lys | Arg ; Gln ; Glu |
| Met | Leu ; Ile |
| Phe | Met ; Leu ; Tyr |
| Ser | Thr |
| Thr | Ser |
| Trp | Tyr |
| Tyr | Trp ; Phe |
| Val | Ile; Leu |

"Funktionale Äquivalente" im obigen Sinne sind auch "Präkursoren" der beschriebenen Polypeptide sowie "funktionale Derivate" und "Salze" der Polypeptide.

"Präkursoren" sind dabei natürliche oder synthetische Vorstufen der Polypeptide mit oder ohne der gewünschten biologischen Aktiviät.

Unter dem Ausdruck "Salze" versteht man sowohl Salze von Carboxylgruppen als auch Säureadditionssalze von Aminogruppen der erfindungsgemäßen Proteinmoleküle. Salze von Carboxylgruppen können in an sich bekannter Weise hergestellt werden und umfassen anorganische Salze, wie zum Beispiel Natrium-, Calcium-, Ammonium-, Eisen- und Zinksalze, sowie Salze mit organischen Basen, wie zum Beispiel Aminen, wie Triethanolamin, Arginin, Lysin, Piperidin und dergleichen. Säureadditionssalze, wie zum Beispiel Salze mit Mineralsäuren, wie Salzsäure oder Schwefelsäure und Salze mit organischen Säuren, wie Essigsäure und Oxalsäure sind ebenfalls Gegenstand der Erfindung.

"Funktionale Derivate" erfindungsgemäßer Polypeptide können an funktionellen Aminosäure-Seitengruppen oder an deren N- oder C-terminalen Ende mit Hilfe bekannter Techniken ebenfalls hergestellt werden: Derartige Derivate umfassen beispielsweise aliphatische Ester von Carbonsäuregruppen, Amide von Carbonsäuregruppen, erhältlich durch Umsetzung mit Ammoniak oder mit einem primären oder sekundären Amin; N-Acylderivate freier Aminogruppen, hergestellt durch Umsetzung mit Acylgruppen; oder O-Acylderivate freier Hydroxygruppen, hergestellt durch Umsetzung mit Acylgruppen.

"Funktionale Äquivalente" umfassen natürlich auch Polypeptide welche aus anderen Organismen zugänglich sind, sowie natürlich vorkommende Varianten. Beispielsweise lassen sich durch Sequenzvergleich Bereiche homologer Sequenzregionen festlegen und in Anlehnung an die konkreten Vorgaben der Erfindung äquivalente Enzyme ermitteln.

"Funktionale Äquivalente" umfassen ebenfalls Fragmente, vorzugsweise einzelne Domänen oder Sequenzmotive, der erfindungsgemäßen Polypeptide, welche z.B. die gewünschte biologische Funktion aufweisen.

"Funktionale Äquivalente" sind außerdem Fusionsproteine, welche eine der oben genannten Polypeptidsequenzen oder davon abgeleitete funktionale Äquivalente und wenigstens eine weitere, davon funktionell verschiedene, heterologe Sequenz in funktioneller N- oder C-terminaler Verknüpfung (d.h. ohne gegenseitigen wesentliche funktionelle Beeinträchtigung der Fusionsproteinteile) aufweisen. Nichtlimitierende Beispiele für derartige heterologe Sequenzen sind z.B. Signalpeptide, Histidin-Anker oder Enzyme.

Erfindungsgemäß mit umfasste "funktionale Äquivalente" sind Homologe zu den konkret offenbarten Proteinen. Diese besitzen wenigstens 60 %, vorzugsweise wenigstens 75% insbesondere wenigstens 85 %, wie z.B. 90, 91, 92, 93, 94, 95, 96, 97,98 oder 99%, Homologie zu einer der konkret offenbarten Aminosäuresequenzen, berechnet nach dem Algorithmus von Pearson und Lipman, Proc. Natl. Acad, Sci. (USA) 85(8), 1988, 2444-2448. Eine prozentuale Homologie eines erfindungsgemäßen homologen Polypeptids bedeutet insbesondere prozentuale Identität der Aminosäurereste bezogen auf die Gesamtlänge einer der hierin konkret beschriebenen Aminosäuresequenzen.

Im Falle einer möglichen Proteinglykosylierung umfassen erfindungsgemäße "funktionale Äquivalente" Proteine des oben bezeichneten Typs in deglykosylierter bzw. glykosylierter Form sowie durch Veränderung des Glykosylierungsmusters erhältliche abgewandelte Formen.

Homologe der erfindungsgemäßen Proteine oder Polypeptide können durch Mutagenese erzeugt werden, z.B. durch Punktmutation, Verlängerung oder Verkürzung des Proteins.

Homologe der erfindungsgemäßen Proteine können durch Screening kombinatorischer Banken von Mutanten, wie z.B. Verkürzungsmutanten, identifiziert werden. Beispielsweise kann eine variegierte Bank von Protein-Varianten durch kombinatorische Mutagenese auf Nukleinsäureebene erzeugt werden, wie z.B. durch enzymatisches Ligieren eines Gemisches synthetischer Oligonukleotide. Es gibt eine Vielzahl von Verfahren, die zur Herstellung von Banken potentieller Homologer aus einer degenerierten Oligonukleotidsequenz verwendet werden können. Die chemische Synthese einer degenerierten Gensequenz kann in einem DNA-Syntheseautomaten durchgeführt werden, und das synthetische Gen kann dann in einen geeigneten Expressionsvektor ligiert werden. Die Verwendung eines degenerierten Gensatzes ermöglicht die Bereitstellung sämtlicher Sequenzen in einem Gemisch, die den gewünschten Satz an potentiellen Proteinsequenzen kodieren. Verfahren zur Synthese degenerierter Oligonukleotide sind dem Fachmann bekannt (z.B. Narang, S.A. (1983) Tetrahedron 39:3; Itakura et al. (1984) Annu. Rev. Biochem. 53:323; Itakura et al., (1984) Science 198:1056; Ike et al. (1983) Nucleic Acids Res. 11:477).

Im Stand der Technik sind mehrere Techniken zum Screening von Genprodukten kombinatorischer Banken, die durch Punktmutationen oder Verkürzung hergestellt worden sind, und zum Screening von cDNA-Banken auf Genprodukte mit einer ausgewählten Eigenschaft bekannt. Diese Techniken lassen sich an das schnelle Screening der Genbanken anpassen, die durch kombinatorische Mutagenese erfindungsgemäßer Homologer erzeugt worden sind. Die am häufigsten verwendeten Techniken zum Screening großer Genbanken, die einer Analyse mit hohem Durchsatz unterliegen, umfassen das Klonieren der Genbank in replizierbare Expressionsvektoren, Transformieren der geeigneten Zellen mit der resultierenden Vektorenbank und Exprimieren der kombinatorischen Gene unter Bedingungen, unter denen der Nachweis der gewünschten Aktivität die Isolation des Vektors, der das Gen kodiert, dessen Produkt nachgewiesen wurde, erleichtert. Recursive-Ensemble-Mutagenese (REM), eine Technik, die die Häufigkeit funktioneller Mutanten in den Banken vergrößert, kann in Kombination mit den Screeningtests verwendet werden, um Homologe zu identifizieren (Arkin und Yourvan (1992) PNAS 89:7811-7815; Delgrave et al. (1993) Protein Engineering 6(3):327-331).

### 3.2 Kodierende Nukleinsäuresequenzen

Gegenstand der Erfindung sind auch Nukleinsäuresequenzen, die für ein Enzym mit Proteinase-Aktivität kodieren. Bevorzugt sind Nukleinsäuresequenzen umfassend eine Sequenz gemäß SEQ ID NO:1; oder eine von der Aminosäuresequenz gemäß SEQ ID NO:: 2 abgeleitete Nukleinsäuresequenz.

Alle hierin erwähnten Nukleinsäuresequenzen (einzel- und doppelsträngige DNA- und RNA-Sequenzen, wie z.B. cDNA und mRNA) sind in an sich bekannter Weise durch chemische Synthese aus den Nukleotidbausteinen, wie beispielsweise durch Fragmentkondensation einzelner überlappender, komplementärer Nukleinsäurebausteine der Doppelhelix herstellbar. Die chemische Synthese von Oligonukleotiden kann beispielsweise, in bekannter Weise, nach der Phosphoamiditmethode (Voet, Voet, 2. Auflage, Wiley Press New York, Seiten 896-897) erfolgen. Die Anlagerung synthetischer Oligonukleotide und Auffüllen von Lücken mit Hilfe des Klenow-Fragmentes der DNA-Polymerase und Ligationsreaktionen sowie allgemeine Klonierungsverfahren werden in Sambrook et al. (1989), Molecular Cloning: A laboratory manual, Cold Spring Harbor Laboratory Press, beschrieben.

Gegenstand der Erfindung sind auch Nukleinsäuresequenzen (einzel- und doppelsträngige DNA- und RNA-Sequenzen, wie z.B. cDNA und mRNA), kodierend für eines der obigen Polypeptide und deren funktionalen Äquivalenten, welche z.B. unter Verwendung künstlicher Nukleotidanaloga zugänglich sind.

Die Erfindung betrifft sowohl isolierte Nukleinsäuremoleküle, welche für erfindungsgemäße Polypeptide bzw. Proteine oder biologisch aktive Abschnitte davon kodieren, als auch Nukleinsäurefragmente, die z.B. zur Verwendung als Hybridisierungssonden oder Primer zur Identifizierung oder Amplifizierung von erfindungsgemäßer kodierenden Nukleinsäuren verwendet werden können.

Die erfindungsgemäßen Nukleinsäuremoleküle können zudem untranslatierte Sequenzen vom 3'- und/oder 5'-Ende des kodierenden Genbereichs enthalten

Die Erfindung umfasst weiterhin die zu den konkret beschriebenen Nukleotidsequenzen komplementären Nukleinsäuremoleküle oder einen Abschnitt davon.

Die erfindungsgemäßen Nukleotidsequenzen ermöglichen die Erzeugung von Sonden und Primern, die zur Identifizierung und/oder Klonierung von homologer Sequenzen in anderen Zelltypen und Organismen verwendbar sind. Solche Sonden bzw. Primer umfassen gewöhnlich einen Nukleotidsequenzbereich, der unter "stringenten" Bedingungen (siehe unten) an mindestens etwa 12, vorzugsweise mindestens etwa 25, wie z.B. etwa 40, 50 oder 75 aufeinanderfolgende Nukleotide eines Sense-Stranges einer erfindungsgemäßen Nukleinsäuresequenz oder eines entsprechenden Antisense-Stranges hybridisiert.

Ein "isoliertes" Nukleinsäuremolekül wird von anderen Nukleinsäuremolekülen abgetrennt, die in der natürlichen Quelle der Nukleinsäure zugegen sind und kann überdies im wesentlichen frei von anderem zellulären Material oder Kulturmedium sein, wenn es durch rekombinante Techniken hergestellt wird, oder frei von chemischen Vorstufen oder anderen Chemikalien sein, wenn es chemische synthetisiert wird.

Ein erfindungsgemäßes Nukleinsäuremolekül kann mittels molekularbiologischer Standard-Techniken und der erfindungsgemäß bereitgestellten Sequenzinformation isoliert werden. Beispielsweise kann cDNA aus einer geeigneten cDNA-Bank isoliert werden, indem eine der konkret offenbarten vollständigen Sequenzen oder ein Abschnitt davon als Hybridisierungssonde und Standard-Hybridisierungstechniken (wie z.B. beschrieben in Sambrook, J., Fritsch, E.F. und Maniatis, T. Molecular Cloning: A Laboratory Manual. 2. Aufl., Cold Spring Harbor Laboratory, Cold Spring Harbor Laboratory Press, Cold Spring Harbor, NY, 1989) verwendet werden. Überdies lässt sich ein Nukleinsäuremolekül, umfassend eine der offenbarten Sequenzen oder ein Abschnitt davon, durch Polymerasekettenreaktion isolieren, wobei die Oligonukleotidprimer, die auf der Basis dieser Sequenz erstellt wurden, verwendet werden. Die so amplifizierte Nukleinsäure kann in einen geeigneten Vektor kloniert werden und durch DNA-Sequenzanalyse charakterisiert werden. Die erfindungsgemäßen Oligonukleotide können ferner durch Standard-Syntheseverfahren, z.B. mit einem automatischen DNA-Synthesegerät, hergestellt werden.

Erfindungsgemäße Nukleinsäuresequenzen, wie SEQ ID No: 1 oder Derivate davon, Homologe oder Teile dieser Sequenzen, lassen sich beispielsweise mit üblichen Hybridisierungsverfahren oder der PCR-Technik aus anderen Bakterien, z.B. über genomische oder cDNA-Banken, isolieren. Diese DNA-Sequenzen hybridisieren unter Standardbedingungen mit den erfindungsgemäßen Sequenzen.

Unter "hybridisieren" versteht man die Fähigkeit eines Poly- oder Oligonukleotids an eine nahezu komplementäre Sequenz unter Standardbedingungen zu binden, während unter diesen Bedingungen unspezifische Bindungen zwischen nicht-komplementären Partnern unterbleiben. Dazu können die Sequenzen zu 90-100% komplementär sein. Die Eigenschaft komplementärer Sequenzen, spezifisch aneinander binden zu können, macht man sich beispielsweise in der Northern- oder Southern-Blot-Technik oder bei der Primerbindung in PCR oder RT-PCR zunutze.

Zur Hybridisierung werden vorteilhaft kurze Oligonukleotide der konservierten Bereiche verwendet. Es können aber auch längere Fragmente der erfindungsgemäßen Nukleinsäuren oder die vollständigen Sequenzen für die Hybridisierung verwendet werden. Je nach der verwendeten Nukleinsäure (Oligonukleotid, längeres Fragment oder vollständige Sequenz) oder je nachdem welche Nukleinsäureart DNA oder RNA für die Hybridisierung verwendet werden, variieren diese Standardbedingungen. So liegen beispielsweise die Schmelztemperaturen für DNA:DNA-Hybride ca 10 °C niedriger als die von DNA:RNA-Hybriden gleicher Länge.

Unter Standardbedingungen sind beispielsweise je nach Nukleinsäure Temperaturen zwischen 42 und 58 °C in einer wäßrigen Pufferlösung mit einer Konzentration zwischen 0,1 bis 5 x SSC (1 X SSC = 0,15 M NaCl, 15 mM Natriumcitrat, pH 7,2) oder zusätzlich in Gegenwart von 50% Formamid wie beispielsweise 42 °C in 5 x SSC, 50% Formamid zu verstehen. Vorteilhafterweise liegen die Hybridisierungsbedingungen für DNA:DNA-Hybride bei 0,1 x SSC und Temperaturen zwischen etwa 20 °C bis 45 °C, bevorzugt zwischen etwa 30 °C bis 45 °C. Für DNA:RNA-Hybride liegen die Hybridisierungsbedingungen vorteilhaft bei 0,1 x SSC und Temperaturen zwischen etwa 30 °C bis 55 °C, bevorzugt zwischen etwa 45 °C bis 55 °C. Diese angegebenen Temperaturen für die Hybridisierung sind beispielhaft kalkulierte Schmelztemperaturwerte für eine Nukleinsäure mit einer Länge von ca. 100 Nukleotiden und einem G + C-Gehalt von 50 % in Abwesenheit von Formamid. Die experimentellen Bedingungen für die DNA-Hybridisierung sind in einschlägigen Lehrbüchern der Genetik, wie beispielsweise Sambrook et al., "Molecular Cloning", Cold Spring Harbor Laboratory, 1989, beschrieben und lassen sich nach dem Fachmann bekannten Formeln beispielsweise abhängig von der Länge der Nukleinsäuren, der Art der Hybride oder dem G + C-Gehalt berechnen. Weitere Informationen zur Hybridisierung kann der Fachmann folgenden Lehrbüchern entnehmen: Ausubel et al. (eds), 1985, Current Protocols in Molecular Biology, John Wiley & Sons, New York; Hames and Higgins (eds), 1985, Nucleic Acids Hybridization: A Practical Approach, IRL Press at Oxford University Press, Oxford; Brown (ed), 1991, Essential Molecular Biology: A Practical Approach, IRL Press at Oxford University Press, Oxford.

Die "Hybridisierung" kann insbesondere unter stringenten Bedingungen erfolgen. Solche Hybridisierungsbedingungen sind beispielsweise bei Sambrook, J., Fritsch, E.F., Maniatis, T., in: Molecular Cloning (A Laboratory Manual), 2. Auflage, Cold Spring Harbor Laboratory Press, 1989, Seiten 9.31-9.57 oder in Current Protocols in Molecular Biology, John Wiley & Sons, N.Y. (1989), 6.3.1-6.3.6 beschrieben.

Unter "stringenten" Hybridisierungs-Bedingungen werden insbesondere verstanden: Die Inkubation bei 42°C über Nacht in einer Lösung bestehend aus 50 % Formamid, 5 x SSC (750 mM NaCl, 75 mM Tri-Natrium Citrat), 50 mM Natrium Phosphat (pH7,6), 5x Denhardt Lösung, 10% Dextransulfat und 20 g/ml denaturierte, gescheerte Lachsspermien-DNA, gefolgt von einem Waschschritt der Filter mit 0,1x SSC bei 65°C.

Gegenstand der Erfindung sind auch Derivate der konkret offenbarten oder ableitbaren Nukleinsäuresequenzen.

So können weitere erfindungsgemäße Nukleinsäuresequenzen z.B. von SEQ ID NO:1 abgeleitet sein und sich davon durch Addition, Substitution, Insertion oder Deletion einzelner oder mehrerer Nukleotide unterscheiden, aber weiterhin für Polypeptide mit dem gewünschten Eigenschaftsprofil kodieren.

Erfindungsgemäß umfasst sind auch solche Nukleinsäuresequenzen, die sogenannte stumme Mutationen umfassen oder entsprechend der Codon-Nutzung eins speziellen Ursprungs- oder Wirtsorganismus, im Vergleich zu einer konkret genannten Sequenz verändert sind, ebenso wie natürlich vorkommende Varianten, wie z.B. Spleißvarianten oder Allelvarianten, davon.

Gegenstand sind ebenso durch konservative Nukleotidsubstutionen (d.h. die betreffende Aminosäure wird durch eine Aminosäure gleicher Ladung, Größe, Polarität und/oder Löslichkeit ersetzt) erhältliche Sequenzen.

Gegenstand der Erfindung sind auch die durch Sequenzpolymorphismen von den konkret offenbarten Nukleinsäuren abgeleiteten Moleküle. Diese genetischen Polymorphismen können zwischen Individuen innerhalb einer Population aufgrund der natürlichen Variation existieren. Diese natürlichen Variationen bewirken üblicherweise eine Varianz von 1 bis 5 % in der Nukleotidsequenz eines Gens.

Unter Derivaten der erfindungsgemäßen Nukleinsäuresequenz mit der Sequenz SEQ ID NO: 1 sind beispielsweise Allelvarianten zu verstehen, die mindestens 60 % Homologie auf der abgeleiteten Aminosäureebene, bevorzugt mindestens 80 % Homologie, ganz besonders bevorzugt mindestens 90 % Homologie über den gesamten Sequenzbereich aufweisen (bezüglich Homologie auf Aminosäureebene sei auf obige Ausführungen zu den Polypeptiden verwiesen auf). Über Teilbereiche der Sequenzen können die Homologien vorteilhaft höher liegen.

Weiterhin sind unter Derivate auch Homologe der erfindungsgemäßen Nukleinsäuresequenzen, insbesondere der SEQ ID NO: 1, beispielsweise pilzliche oder bakterielle Homologe, verkürzte Sequenzen, Einzelstrang-DNA oder RNA der kodierenden und nichtkodierenden DNA-Sequenz, zu verstehen. So besitzen z.B. Homologe zur der SEQ ID NO: 1 auf DNA-Ebene eine Homologie von mindestens 40 %, bevorzugt von mindestens 60 %, besonders bevorzugt von mindestens 70 %, ganz besonders bevorzugt von mindestens 80 % über den gesamten in SEQ ID NO: 1 angegebenen DNA-Bereich.

Außerdem sind unter Derivaten beispielsweise Fusionen mit Promotoren zu verstehen. Die Promotoren, die den angegebenen Nukleotidsequenzen vorgeschalten sind, können durch wenigstens einen Nukleotidaustausch, wenigstens eine Insertionen, Inversionen und/oder Deletionen verändert sein, ohne dass aber die Funktionalität bzw. Wirksamkeit der Promotoren beeinträchtigt sind. Des weiteren können die Promotoren durch Veränderung ihrer Sequenz in ihrer Wirksamkeit erhöht oder komplett durch wirksamere Promotoren auch artfremder Organismen ausgetauscht werden.

### 3.3 Erfindungsgemäße Konstrukte

Gegenstand der Erfindung sind außerdem Expressionskonstrukte, enthaltend unter der genetischen Kontrolle regulativer Nukleinsäuresequenzen eine für ein erfindungsgemäßes Polypeptid kodierende Nukleinsäuresequenz; sowie Vektoren, umfassend wenigstens eines dieser Expressionskonstrukte.

Unter einer "Expressionseinheit" wird erfindungsgemäß eine Nukleinsäure mit Expressionsaktivität verstanden, die einen Promotor, wie hierein definiert umfasst, und nach funktioneller Verknüpfung mit einer zu exprimierenden Nukleinsäure oder einem Gen, die Expression, also die Transkription und die Translation dieser Nukleinsäure oder dieses Gens reguliert. Man spricht deshalb auch in diesem Zusammenhang von einer "regulativen Nukleinsäuresequenz". Zusätzlich zum Promotor können weitere, regulative Elemente, wie z.B. Enhancer, enthalten sein.

Unter einer "Expressionskassette" oder "Expressionskonstrukt" wird erfindungsgemäß eine Expressionseinheit verstanden, die mit der zu exprimierenden Nukleinsäure oder dem zu exprimierenden Gen funktionell verknüpft ist. Im Gegensatz zu einer Expressionseinheit umfasst eine Expressionskassette somit nicht nur Nukleinsäuresequenzen, welche Transkription und Translation regulieren, sondern auch die Nukleinsäuresequenzen, welche als Folge der Transkription und Translation als Protein exprimiert werden sollen.

Die Begriffe "Expression" oder "Überexpression" beschreiben im Kontext der Erfindung die Produktion bzw. Erhöhung der intrazellulären Aktivität eines oder mehrerer Enzyme in einem Mikroorganismus, die durch die entsprechende DNA kodiert werden. Dazu kann man beispielsweise ein Gen in einen Organismus einbringen, ein vorhandenes Gen durch ein anderes Gen ersetzen, die Kopienzahl des Gens bzw. der Gene erhöhen, einen starken Promotor verwenden oder ein Gen verwenden, das für ein entsprechendes Enzym mit einer hohen Aktivität kodiert und man kann gegebenenfalls diese Maßnahmen kombinieren.

Vorzugsweise umfassen solche erfindungsgemäßen Konstrukte 5'-stromaufwärts von der jeweiligen kodierenden Sequenz einen Promotor und 3'-stromabwärts eine Terminatorsequenz sowie gegebenenfalls weitere übliche regulative Elemente, und zwar jeweils operativ verknüpft mit der kodierenden Sequenz.

Unter einem "Promotor", einer "Nukleinsäure mit Promotoraktivität" oder einer "Promotorsequenz" wird erfindungsgemäß eine Nukleinsäure verstanden, die in funktioneller Verknüpfung mit einer zu transkribierenden Nukleinsäure die Transkription dieser Nukleinsäure reguliert.

Unter einer "funktionellen" oder "operativen" Verknüpfung versteht man in diesem Zusammenhang beispielsweise die sequentielle Anordnung einer der Nukleinsäuren mit Promotoraktivität und einer zu transkribierenden Nukleinsäuresequenz und gegebenenfalls weiterer regulativer Elemente, wie zum Beispiel Nukleinsäuresequenzen, die die Transkription von Nukleinsäuren gewährleisten, sowie zum Beispiel einen Terminator, derart, dass jedes der regulativen Elemente seine Funktion bei der Transkription der Nukleinsäuresequenz erfüllen kann. Dazu ist nicht unbedingt eine direkte Verknüpfung im chemischen Sinne erforderlich. Genetische Kontrollsequenzen, wie zum Beispiel Enhancer-Sequenzen, können ihre Funktion auch von weiter entfernten Positionen oder gar von anderen DNA-Molekülen aus auf die Zielsequenz ausüben. Bevorzugt sind Anordnungen, in denen die zu transkribierende Nukleinsäuresequenz hinter (d.h. am 3'-Ende) der Promotorsequenz positioniert wird, so dass beide Sequenzen kovalent miteinander verbunden sind. Dabei kann der Abstand zwischen der Promotorsequenz und der transgen zu exprimierende Nukleinsäuresequenz geringer als 200 Basenpaare, oder kleiner als 100 Basenpaare oder kleiner als 50 Basenpaare sein.

Neben Promotoren und Terminator sind als Beispiele weiterer regulativer Elemente zu nennen Targeting-Sequenzen, Enhancer, Polyadenylierungssignale, selektierbare Marker, Amplifikationssignale, Replikationsursprünge und dergleichen. Geeignete regulatorische Sequenzen sind z.B. beschrieben in Goeddel, Gene Expression Technology: Methods in Enzymology 185, Academic Press, San Diego, CA (1990).

Erfindungsgemäße Nukleinsäurekonstrukte umfassen insbesondere Sequenz SEQ ID NO: 1 oder Derivate und Homologe davon, sowie die von SEQ ID NO: 2 ableitbaren Nukleinsäuresequenzen, die mit einem oder mehreren Regulationssignalen vorteilhafterweise zur Steuerung, z.B. Erhöhung, der Genexpression operativ oder funktionell verknüpft wurden.

Zusätzlich zu diesen Regulationssequenzen kann die natürliche Regulation dieser Sequenzen vor den eigentlichen Strukturgenen noch vorhanden sein und gegebenenfalls genetisch verändert worden sein, so dass die natürliche Regulation ausgeschaltet und die Expression der Gene erhöht wurde. Das Nukleinsäurekonstrukt kann aber auch einfacher aufgebaut sein, das heißt es wurden keine zusätzlichen Regulationssignale vor die kodierende Sequenz (wie z.B. SEQ ID NO: 1 oder seine Homologen) insertiert und der natürliche Promotor mit seiner Regulation wurde nicht entfernt. Stattdessen wird die natürliche Regulationssequenz so mutiert, dass keine Regulation mehr erfolgt und die Genexpression gesteigert wird.

Ein bevorzugtes Nukleinsäurekonstrukt enthält vorteilhafterweise auch eine oder mehrere der schon erwähnten "Enhancer" Sequenzen, funktionell verknüpft mit dem Promotor, die eine erhöhte Expression der Nukleinsäuresequenz ermöglichen. Auch am 3'-Ende der DNA-Sequenzen können zusätzliche vorteilhafte Sequenzen inseriert werden, wie weitere regulatorische Elemente oder Terminatoren. Die erfindungsgemäßen Nukleinsäuren können in einer oder mehreren Kopien im Konstrukt enthalten sein. Im Konstrukt können noch weitere Marker, wie Antibiotikaresistenzen oder Auxotrophien komplementierende Gene, gegebenenfalls zur Selektion auf das Konstrukt enthalten sein.

Beispiele geeigneter Regulationssequenzen sind in Promotoren wie cos-, tac-, trp-, tet-, trp-tet-, lpp-, lac-, lpp-lac-, lacl^{q-,} T7-, T5-, T3-, gal-, trc-, ara-, rhaP (rhaP_{BAD})SP6-, lambda-P_{R}- oder im lambda-P_{L}-Promotor enthalten, die vorteilhafterweise in gramnegativen Bakterien Anwendung finden. Weitere vorteilhafte Regulationssequenzen sind beispielsweise in den gram-positiven Promotoren amy und SPO2, in den Hefe- oder Pilzpromotoren ADC1, MFalpha , AC, P-60, CYC1, GAPDH, TEF, rp28, ADH enthalten. Es können auch künstliche Promotoren für die Regulation verwendet werden.

Das Nukleinsäurekonstrukt wird zur Expression in einem Wirtsorganismus vorteilhafterweise in einen Vektor, wie beispielsweise einem Plasmid oder einem Phagen inseriert, der eine optimale Expression der Gene im Wirt ermöglicht. Unter Vektoren sind außer Plasmiden und Phagen auch alle anderen dem Fachmann bekannten Vektoren, also z.B. Viren, wie SV40, CMV, Baculovirus und Adenovirus, Transposons, IS-Elemente, Phasmide, Cosmide, und lineare oder zirkuläre DNA zu verstehen. Diese Vektoren können autonom im Wirtsorganismus repliziert oder chromosomal repliziert werden. Diese Vektoren stellen eine weitere Ausgestaltung der Erfindung dar.

Geeignete Plasmide sind beispielsweise in *E. coli* pLG338, pACYC184, pBR322, pUC18, pUC19, pKC30, pRep4, pHS1, pKK223-3, pDHE19.2, pHS2, pPLc236, pMBL24, pLG200, pUR290, pIN-III¹¹³-B1, λgt11 oder pBdCl, in *Streptomyces* plJ101, pIJ364, pIJ702 oder pIJ361, in *Bacillus* pUB110, pC194 oder pBD214, in *Corynebacterium* pSA77 oder pAJ667, in Pilzen pALS1, pIL2 oder pBB116, in Hefen 2alphaM, pAG-1, YEp6, YEp13 oder pEMBLYe23 oder in Pflanzen pLGV23, pGHlac⁺, pBIN19, pAK2004 oder pDH51. Die genannten Plasmide stellen eine kleine Auswahl der möglichen Plasmide dar. Weitere Plasmide sind dem Fachmann wohl bekannt und können beispielsweise aus dem Buch Cloning Vectors (Eds. Pouwels P. H. et al. Elsevier, Amsterdam-New York-Oxford, 1985 , ISBN 0 444 904018) entnommen werden.

In einer weiteren Ausgestaltungsform des Vektors kann der das erfindungsgemäße Nukleinsäurekonstrukt oder die erfindungsgemäße Nukleinsäure enthaltende Vektor auch vorteilhafterweise in Form einer linearen DNA in die Mikroorganismen eingeführt werden und über heterologe oder homologe Rekombination in das Genom des Wirtsorganismus integriert werden. Diese lineare DNA kann aus einem linearisierten Vektor wie einem Plasmid oder nur aus dem Nukleinsäurekonstrukt oder der erfindungsgemäßen Nukleinsäure bestehen.

Für eine optimale Expression heterologer Gene in Organismen ist es vorteilhaft die Nukleinsäuresequenzen entsprechend des im Organismus verwendeten spezifischen "codon usage" zu verändern. Der "codon usage" läßt sich anhand von Computerauswertungen anderer, bekannter Gene des betreffenden Organismus leicht ermitteln.

Die Herstellung einer erfindungsgemäßen Expressionskassette erfolgt durch Fusion eines geeigneten Promotors mit einer geeigneten kodierenden Nukleotidsequenz sowie einem Terminator- oder Polyadenylierungssignal. Dazu verwendet man gängige Rekombinations- und Klonierungstechniken, wie sie beispielsweise in T. Maniatis, E.F. Fritsch und J. Sambrook, Molecular Cloning: A Laboratory Manual, Cold Spring Harbor Laboratory, Cold Spring Harbor, NY (1989) sowie in T.J. Silhavy, M.L. Berman und L.W. Enquist, Experiments with Gene Fusions, Cold Spring Harbor Laboratory, Cold Spring Harbor, NY (1984) und in Ausubel, F.M. et al., Current Protocols in Molecular Biology, Greene Publishing Assoc. and Wiley Interscience (1987) beschrieben sind.

Das rekombinante Nukleinsäurekonstrukt bzw. Genkonstrukt wird zur Expression in einem geeigneten Wirtsorganismus vorteilhafterweise in einen wirtsspezifischen Vektor insertiert, der eine optimale Expression der Gene im Wirt ermöglicht. Vektoren sind dem Fachmann wohl bekannt und können beispielsweise aus "Cloning Vectors" (Pouwels P. H. et al., Hrsg, Elsevier, Amsterdam-New York-Oxford, 1985) entnommen vierden.

### 3.4 Erfindungsgemäß brauchbare Wirte

Je nach Zusammenhang kann unter dem Begriff "Mikroorganismus" der Ausgangsmikroorganismus (Wildtyp) oder ein erfindungsgemäßer, genetisch veränderter Mikroorganismus oder beides verstanden werden.

Unter dem Begriff "Wildtyp" wird erfindungsgemäß der entsprechende Ausgangsmikroorganismus verstanden und muss nicht zwingend einem natürlich vorkommenden Organismus entsprechen.

Mit Hilfe der erfindungsgemäßen Vektoren sind rekombinante Mikroorganismen herstellbar, welche beispielsweise mit wenigstens einem erfindungsgemäßen Vektor transformiert sind und zur Produktion der erfindungsgemäßen Polypeptide eingesetzt werden können. Vorteilhafterweise werden die oben beschriebenen erfindungsgemäßen rekombinanten Konstrukte in ein geeignetes Wirtssystem eingebracht und exprimiert. Dabei werden vorzugsweise dem Fachmann bekannte geläufige Klonierungs- und Transfektionsmethoden, wie beispielsweise Co-Präzipitation, Protoplastenfusion, Elektroporation, retrovirale Transfektion und dergleichen, verwendet, um die genannten Nukleinsäuren im jeweiligen Expressionssystem zur Expression zu bringen. Geeignete Systeme werden beispielsweise in Current Protocols in Molecular Biology, F. Ausubel et al., Hrsg., Wiley Interscience, New York 1997, oder Sambrook et al. Molecular Cloning: A Laboratory Manual. 2. Aufl., Cold Spring Harbor Laboratory, Cold Spring Harbor Laboratory Press, Cold Spring Harbor, NY, 1989 beschrieben.

Als rekombinante Wirtsorganismen für die erfindungsgemäße Nukleinsäure oder dem Nukleinsäurekonstrukt kommen prinzipiell alle prokaryontischen oder eukaryontischen Organismen in Frage. Vorteilhafterweise werden als Wirtsorganismen Mikroorganismen wie Bakterien, Pilze oder Hefen verwendet. Vorteilhaft werden gram-positive oder gram-negative Bakterien, bevorzugt Bakterien der Familien Enterobacteriaceae, *Pseudomonadaceae, Rhizobiaceae, Streptomycetaceae* oder *Nocardiaceae*, besonders bevorzugt Bakterien der Gattungen *Escherichia, Pseudomonas, Streptomyces, Nocardia, Burkholderia, Salmonella, Agrobacterium* oder *Rhodococcus* verwendet. Ganz besonders bevorzugt ist die Gattung und Art *Escherichia coli.* Weitere vorteilhafte Bakterien sind darüber hinaus in der Gruppe der alpha-Proteobacterien, beta-Proteobacterien oder gamma-Proteobacterien zu finden.

Der Wirtsorganismus oder die Wirtsorganismen gemäß der Erfindung enthalten dabei vorzugsweise mindestens eine der in dieser Erfindung beschriebenen Nukleinsäuresequenzen, Nukleinsäurekonstrukte oder Vektoren, die für ein Enzym mit Proteaseaktivität gemäß obiger Definition kodieren.

Die im erfindungsgemäßen Verfahren verwendeten Organismen werden je nach Wirtsorganismus in dem Fachmann bekannter Weise angezogen bzw. gezüchtet. Mikroorganismen werden in der Regel in einem flüssigen Medium, das eine Kohlenstoffquelle meist in Form von Zuckern, eine Stickstoffquelle meist in Form von organischen Stickstoffquellen wie Hefeextrakt oder Salzen wie Ammoniumsulfat, Spurenelemente wie Eisen-, Mangan-, Magnesiumsalze und gegebenenfalls Vitamine enthält, bei Temperaturen zwischen 0 °C und 100 °C, bevorzugt zwischen 10 °C bis 60 °C unter Sauerstoffbegasung angezogen. Dabei kann der pH der Nährflüssigkeit auf einen festen Wert gehalten werden, das heißt während der Anzucht reguliert werden oder nicht. Die Anzucht kann "batch"-weise, "semi batch"-weise oder kontinuierlich erfolgen. Nährstoffe können zu beginn der Fermentation vorgelegt oder semikontinuierlich oder kontinuierlich nachgefüttert werden. Das Keton kann direkt zur Anzucht gegeben werden oder vorteilhaft nach Anzucht. Die Enzyme können nach dem in den Beispielen beschriebenen Verfahren aus den Organismen isoliert werden oder als Rohextrakt für die Reaktion verwendet werden.

Erfindungsgemäß brauchbare Wirte sind insbesondere Bakterien der Gattung Burkholderia, insbesondere der Spezies *Burkholderia glumae.* Als nichtlimitierendes Beispiel kann der allgemein zugängliche Stamm DSM No: 9512 von *Burkholderia glumae* (Synonym: *Pseudomonas glumae*) genannt werden.

### 3.5 Rekombinante Herstellung der Protease:

Gegenstand der Erfindung sind weiterhin Verfahren zur rekombinanten Herstellung erfindungsgemäße Polypeptide oder funktioneller, biologisch aktiver Fragmente davon, wobei man einen Polypeptide-produzierenden Mikroorganismus kultiviert, gegebenenfalls die Expression der Polypeptide induziert und diese aus der Kultur isoliert. Die Polypeptide können so auch in großtechnischem Maßstab produziert werden, falls dies erwünscht ist.

Die erfindungsgemäß hergestellten Mikroorganismen können kontinuierlich oder diskontinuierlich im batch- Verfahren (Satzkultivierung) oder im fed batch (Zulaufverfahren) oder repeated fed batch Verfahren (repetitives Zulaufverfahren) kultiviert werden. Eine Zusammenfassung über bekannte Kultivierungsmethoden ist im Lehrbuch von Chmiel (Bioprozeßtechnik 1. Einführung in die Bioverfahrenstechnik (Gustav Fischer Verlag, Stuttgart, 1991)) oder im Lehrbuch von Storhas (Bioreaktoren und periphere Einrichtungen (Vieweg Verlag, Braunschweig/Wiesbaden, 1994)) zu finden.

Das zu verwendende Kulturmedium hat in geeigneter Weise den Ansprüchen der jeweiligen Stämme zu genügen. Beschreibungen von Kulturmedien verschiedener Mikroorganismen sind im Handbuch "Manual of Methods für General Bacteriology" der American Society für Bacteriology (Washington D. C., USA, 1981) enthalten.

Diese erfindungsgemäß einsetzbaren Medien umfassen gewöhnlich eine oder mehrere Kohlenstoffquellen, Stickstoffquellen, anorganische Salze, Vitamine und/oder Spurenelemente.

Bevorzugte Kohlenstoffquellen sind Zucker, wie Mono-, Di- oder Polysaccharide. Sehr gute Kohlenstoffquellen sind beispielsweise Glucose, Fructose, Mannose, Galactose, Ribose, Sorbose, Ribulose, Lactose, Maltose, Saccharose, Raffinose, Stärke oder Cellulose. Man kann Zucker auch über komplexe Verbindungen, wie Melassen, oder andere Nebenprodukte der Zucker-Raffinierung zu den Medien geben. Es kann auch vorteilhaft sein, Gemische verschiedener Kohlenstoffquellen zuzugeben. Andere mögliche Kohlenstoffquellen sind Öle und Fette wie z. B. Sojaöl. Sonnenblumenöl. Erdnußöl und Kokosfett, Fettsäuren wie z. B. Palmitinsäure, Stearinsäure oder Linolsäure, Alkohole wie z. B. Glycerin, Methanol oder Ethanol und organische Säuren wie z. B. Essigsäure oder Milchsäure.

Stickstoffquellen sind gewöhnlich organische oder anorganische Stickstoffverbindungen oder Materialien, die diese Verbindungen enthalten. Beispielhafte Stickstoffquellen umfassen Ammoniak-Gas oder Ammoniumsalze, wie Ammoniumsulfat, Ammoniumchlorid, Ammoniumphosphat, Ammoniumcarbonat oder Ammoniumnitrat, Nitrate, Harnstoff, Aminosäuren oder komplexe Stickstoffquellen, wie Maisquellwasser, Sojamehl, Sojaprotein, Hefeextrakt, Fleischextrakt und andere. Die Stickstoffquellen können einzeln oder als Mischung verwendet werden.

Anorganische Salzverbindungen, die in den Medien enthalten sein können, umfassen die Chlorid-, Phosphor- oder Sulfatsalze von Calcium, Magnesium, Natrium, Kobalt, Molybdän, Kalium, Mangan, Zink, Kupfer und Eisen.

Als Schwefelquelle können anorganische schwefelhaltige Verbindungen wie beispielsweise Sulfate, Sulfite, Dithionite, Tetrathionate, Thiosulfate, Sulfide aber auch organische Schwefelverbindungen, wie Mercaptane und Thiole, verwendet werden.

Als Phosphorquelle können Phosphorsäure, Kaliumdihydrogenphosphat oder Dikaliumhydrogenphosphat oder die entsprechenden Natrium haltigen Salze verwendet werden.

Chelatbildner können zum Medium gegeben werden, um die Metallionen in Lösung zu halten. Besonders geeignete Chelatbildner umfassen Dihydroxyphenole, wie Catechol oder Protocatechuat, oder organische Säuren, wie Citronensäure.

Die erfingdungsgemäß eingesetzten Fermentationsmedien enthalten üblicherweise auch andere Wachstumsfaktoren, wie Vitamine oder Wachstumsförderer, zu denen beispielsweise Biotin, Riboflavin, Thiamin, Folsäure, Nikotirisäure, Panthothenat und Pyridoxin gehören. Wachstumsfaktoren und Salze stammen häufig von komplexen Medienkomponenten, wie Hefeextrakt, Melassen, Maisquellwasser und dergleichen. Dem Kulturmedium können überdies geeignete Vorstufen zugesetzt werden. Die genaue Zusammensetzung der Medienverbindungen hängt stark vom jeweiligen Experiment ab und wird für jeden spezifische Fall individuell entschieden. Information über die Medienoptimierung ist erhältlich aus dem Lehrbuch "Applied Microbiol. Physiology, A Practical Approach" (Hrsg. P.M. Rhodes, P.F. Stanbury, IRL Press (1997) S. 53-73, ISBN 0 19 963577 3). Wachstumsmedien lassen sich auch von kommerziellen Anbietern beziehen, wie Standard 1 (Merck) oder BHI (Brain heart infusion, DIFCO) und dergleichen.

Sämtliche Medienkomponenten werden, entweder durch Hitze (20 min bei 1,5 bar und 121 °C) oder durch Sterilfiltration, sterilisiert. Die Komponenten können entweder zusammen oder nötigenfalls getrennt sterilisiert werden. Sämtliche Medienkomponenten können zu Beginn der Anzucht zugegen sein oder wahlfrei kontinuierlich oder chargenweise hinzugegeben werden.

Die Temperatur der Kultur liegt normalerweise zwischen 15°C und 45°C, vorzugsweise bei 25°C bis 40°C und kann während des Experimentes konstant gehalten oder verändert werden. Der pH-Wert des Mediums sollte im Bereich von 5 bis 8,5, vorzugsweises um 7,0 liegen. Der pH-Wert für die Anzucht läßt sich während der Anzucht durch Zugabe von basische Verbindungen wie Natriumhydroxid, Kaliumhydroxid, Ammoniak bzw. Ammoniakwasser oder saure Verbindungen wie Phosphorsäure oder Schwefelsäure kontrollieren. Zur Kontrolle der Schaumentwicklung können Antischaummittel, wie z. B. Fettsäurepolyglykolester, eingesetzt werden. Zur Aufrechterhaltung der Stabilität von Plasmiden können dem Medium geeignete selektiv wirkende Stoffe, wie z. B. Antibiotika, hinzugefügt werden. Um aerobe Bedingungen aufrechtzuerhalten, werden Sauerstoff oder Sauerstoff-haltige Gasmischungen, wie z. B. Umgebungsluft, in die Kultur eingetragen. Die. Temperatur der Kultur liegt normalerweise bei 20°C bis 45°C und. Die Kultur wird so lange fortgesetzt, bis sich ein Maximum des gewünschten Produktes gebildet hat. Dieses Ziel wird normalerweise innerhalb von 10 Stunden bis 160 Stunden erreicht.

Die Fermentationsbrühe wird anschließend weiterverarbeitet. Je nach Anforderung kann die Biomasse ganz oder teilweise durch Separationsmethoden, wie z. B. Zentrifugation, Filtration, Dekantieren oder einer Kombination dieser Methoden aus der Fermentationsbrühe entfernt oder vollständig in ihr belassen werden.

Die Zellen können auch, falls die Polypeptide nicht in das Kulturmedium sezerniert werden, aufgeschlossen und das Produkt nach bekannten Proteinisolierungsverfahren aus dem Lysat gewonnen. Die Zellen können wahlweise durch hochfrequenten Ultraschall, durch hohen Druck, wie z.B. in einer French-Druckzelle, durch Osmolyse, durch Einwirkung von Detergenzien, lytischen Enzymen oder organischen Lösungsmitteln, durch Homogenisatoren oder durch Kombination mehrerer der aufgeführten Verfahren aufgeschlossen werden.

Eine Aufreinigung der Polypeptide kann mit bekannten, chromatographischen Verfahren erzielt werden, wie Molekularsieb-Chromatographie (Gelfiltration), wie Q-Sepharose-Chromatographie, lonenaustausch-Chromatographie und hydrophobe Chromatographie, sowie mit anderen üblichen Verfahren wie Ultrafiltration, Kristallisation, Aussalzen, Dialyse und nativer Gelelektrophorese. Geeignete Verfahren werden beispielsweise in Cooper, F. G., Biochemische Arbeitsmethoden, Verlag Walter de Gruyter, Berlin, New York oder in Scopes, R., Protein Purification, Springer Verlag, New York, Heidelberg, Berlin beschrieben.

Vorteilhaft kann es sein, zur Isolierung des rekombinanten Proteins Vektorsysteme oder Oligonukleotide zu verwenden, die die cDNA um bestimmte Nukleotidsequenzen verlängern und damit für veränderte Polypeptide oder Fusionsproteine kodieren, die z.B. einer einfacheren Reinigung dienen. Derartige geeignete Modifikationen sind beispielsweise als Anker fungierende sogenannte "Tags", wie z.B. die als Hexa-Histidin-Anker bekannte Modifikation oder Epitope, die als Antigene von Antikörpern erkannt werden können (beschrieben zum Beispiel in Harlow, E. and Lane, D., 1988, Antibodies: A Laboratory Manual. Cold Spring Harbor (N.Y.) Press). Diese Anker können zur Anheftung der Proteine an einen festen Träger, wie z.B. einer Polymermatrix, dienen, die beispielsweise in einer Chromatographiesäule eingefüllt sein kann, oder an einer Mikrotiterplatte oder an einem sonstigen Träger verwendet werden kann.

Gleichzeitig können diese Anker auch zur Erkennung der Proteine verwendet werden. Zur Erkennung der Proteine können außerdem übliche Marker, wie Fluoreszenzfarbstoffe, Enzymmarker, die nach Reaktion mit einem Substrat ein detektierbäres Reaktionsprodukt bilden, oder radioaktive Marker, allein oder in Kombination mit den Ankern zur Derivatisierung der Proteine verwendet werden.

### 3.6 Durchführung des erfindungsgemäßen Verfahrens zur Herstellung von Lipasen mit B. glumae

Im wesentlichen wird die Lipase durch Fermentation von *Burkholderia glumae* hergestellt. In einem speziellen Produktionsmedium wird der Organismus angezogen. Dabei sekretiert *Burkholderia glumae* die Lipase in aktiver Form ins Medium. Am Ende der Fermentation werden die Zellen durch Dekantieren / Zentrifugieren vom Medium getrennt. Der anfallende Rückstand kann getrocknet werden, um Lipase als Trockenformulierung zu erhalten. Es ist weiterhin möglich, die Lipase aus dem Medium durch geeignete chromatographische Verfahren in hochreiner Form zu gewinnen. Schließlich ist auch eine direkte Adsorption aus dem Medium an geeignete Träger durch Immobilisierung möglich. Dabei kommen dem Protein-Fachmann geläufige Standardmethoden zur Anwendung.

Im Übrigen erfolgt die mikrobielle Produktion und Isolierung der extrazellulären Lipase analog zu derjenigen der Protease (vgl. oben).

Die erfindungsgemäß besser zugänglichen Lipasen können beispielsweise zur Herstellung von Feinchemikalien verwendet werden. Dabei wird sie insbesondere zur Gewinnung von optisch aktiven Verbindungen mittels Racematspaltung verwendet. Denkbar ist aber der Einsatz als Katalysatoren zur enantioselektiven Synthese. Schließlich kann das Enzym nicht nur wegen seiner Stereoselektivität, sondern auch wegen seiner Regioselektivität verwendet werden. Mögliche Einsatzgebiete sind die Modifikation von Polymeren oder niedermolekularen Verbindungen, bei denen regioselektiv Esterbindungen gespalten oder geknüpft werden.

Die nachstehenden Beispiele dienen nur der Verdeutlichung der Erfindung. Die für den Fachmann offensichtlichen, zahlreich möglichen Abwandlungen sind erfindungsgemäß ebenfalls umfasst.

### EXPERIMENTELLER TEIL

Die im Rahmen der vorliegenden Erfindung durchgeführten Klonierungsschritte wie z.B. Restriktionsspaltungen, Agarose Gelelektrophorese, Reinigung von DNA-Fragmenten. Transfer von Nukleinsäuren auf Nitrozellulose und Nylonmembranen, Verknüpfen von DNA-Fragmenten, Transformation von E. coli Zellen, Anzucht von Bakterien, Vermehrung von Phagen und Sequenzanalyse rekombinanter DNA wurden wie bei Sambrook et al. (1989) a.a.O. beschrieben durchgeführt.

### 1. Versuchsbeschreibung

### a) Bakterienstämme und Wachstumsbedingungen

*E. coli*. DH5α wurde für die Routine-Klonierungsexperimente verwendet. Dieser Stamm wurde in Luria Bertani-Medium bei 37°C kultiviert (LB-Medium (pH 7,0): 10 g/l NaCl, 10 g/l Bacto-Trypton, 5 g/l Hefeextrakt; Sambrook J. et al. [17]. Ampicillin (100 µg/ml), Tetracyclin (25 µg/ml) oder Chloramphenicol (50 µg/ml) wurden für die Erhaltung des Plasmids eingesetzt. Für die Expressionsuntersuchungen wurden der Wildtypstamm *B. glumae* PG1 (erhalten von Jan Tommassen, Universität Utrecht, Niederlande) und der Produktionsstamm *B. glumae* LU8093 verwendet.

Diese Stämme wurden bei 30 °C in PG-Medium kultiviert, welches pro Liter enthielt: 6 g (NH₄)₂SO₄, 3,5 g KH₂PO₄, 3,5 g K₂HPO₄, 0,02 g CaCl₂, 1 g MgSO₄ x 7 H₂O und 2 g Hefeextrakt, pH 6,5 [6]. Für die Induktion der Lipaseproduktion wurde 1 % (v/v) Olivenöl (Sigma) zugesetzt und für die Erhaltung des Expressionsplasmids wurde Chloramphenicol (200 µg/ml) dem Medium zugesetzt.

### b) Herstellung erfindungsgemäßer Plasmid- und Cosmid-Konstrukte

Allgemeine Methoden der DNA-Manipulation wurden in Übereinstimmung mit Sambrook et al. [17] oder nach Angaben der Hersteller für DNA-modifizierende Enzyme durchgeführt.

Die Konstruktion der genomischen Bibliothek von *B. glumae* PG1 erfolgte im Wesentlichen nach der Beschreibung von Staskawicz et al. [20]. Nach Isolation genomischer DNA aus Über-Nacht-Kulturen wurde die DNA unter Verwendung von Sau3A partiell hydrolysiert. Die DNA-Fragmente wurden durch Elektrophorese über ein 0,5 %iges Agarose-Gel in standardisiertem Tris-Acetat-EDTA-Puffer aufgetrennt und Fragmente mit einer Größe von mehr als 15 kb wurden ausgeschnitten und gereinigt. Das Cosmid pLAFR3 (Staskawicz et al, [20]) wurde in zwei verschiedenen Reaktionen mit den Restriktionsenzymen HindIII und EcoRI hydrolysiert und anschießend dephosphoryliert. In einer zweiten Stufe wurden beide Restriktionsansätze mit BamHI verdaut, um Sau3A-kompatible Enden zu erhalten. Die Ligation der genomischen DNA und der beiden DNA-Fragmente von pLAFR3 wurde über Nacht bei 16 °C unter Verwendung von T4 DNA-Ligase vorgenommen. Schließlich wurden die Cosmide in vitro verpackt und in *E. coli* JM101 transduziert. Da das Cosmid pLAFR3 ein Blau-Weiß-Screening erlaubt, konnten positive Klone auf LB-Agar-Platten, enthaltend.Tetracyclin (25µg/ml), Isopropyl-1-thio-β-*D*-galactopyranosid (IPTG, 250 µg/ml Endkonzentration) und 5 Brom-4-chlor-3-indolyl-β-*D*-galactopyranosid (40-Gal, 40 µg/ml Endkonzentration) identifiziert werden. Insgesamt wurden 9300 Klone selektiert und auf Mikrotiterplatten übertragen, was zu einer 4-fachen Abdeckung des *B. glumae* PG1-Genoms führte.

Die DNA-Fragmente der so erhaltenen Cosmide pLAFRPG5 und pLAFRPG8 zeigten einen Einfluss auf die Lipaseproduktion in *B. glumae.* Diese wurden durch Insertion der EcoRI/HindIII-Fragmente in pBBR1mcs (Kovach, M.E. et al.[30]) subkloniert. Dabei erhielt man die Plasmide pBBRPG 5/1, 5/3, 5/7, 8/1 + 8/3, welche einer DNA-Sequenzierung unterzogen wurden.

Das DNA-Fragment, welches die 179 Aminosäuren der erfindungsgemäßen Protease kodiert, wurde durch Polymerasekettenreaktion unter Verwendung der genomischen DNA von *B. glumae* PG1 als Template amplifiziert. Der dem 5'-Ende der amplifizierten DNA entsprechende Oligonukleotid-Primer Proᵤₚ (CTAGGATCCAATTCGCCTCATC-CAATGCA) (SEQ ID NO: 8) wurde mit einer BamHI-Erkennungssequenz ausgestattet und der zweite Primer Pro_{down} (CGCAAGCTTTCACGCGCCGGCCC) (SEQ ID NO: 9) wurde mit einer Hindlll-Erkennungssequenz ausgestattet. Das amplifizierte DNA-Fragment mit einer Größe von 540 bp wurde in die BamHI/HindIII-Stelle des pBBR1mcs einkloniert, dessen Expression unter der Kontrolle des lac-Promotors stand. Das resultierende Plasmid mit der Bezeichnung pBBRpro (vgl. Figur 2B) wurde in *B. glumae* durch Konjugation überführt. Für die Überexpression in *E. coli* wurde das PCR-Produkt unter Verwendung folgender Primer reamplifiziert: ProᵤₚN (GTACA-TATGCAGCAGCGTGGC) (SEQ ID NO: 10) mit einer Ndel-Erkennungssequenz und Pro_{down}X (ATCTCGAGTCACGCGCCGGCC) (SEQ ID NO: 11) mit einer Xhol-Erkennungssequenz. Das DNA-Fragment wurde anschließend in die NdeI/HindIII-Position von pET22b (Novagen Inc., Madison, WI, USA (1997)) kloniert und stand somit unter der Kontrolle des T7-Promotors und wies einen C-terminalen 6xHis-tag auf, der die Reinigung durch Nickel-Nitrilotriessigsäure Metall-Affinitätschromatographie ermöglicht. Das resultierende Plasmid mit der Bezeichnung pETpro (vgl. Figur 2A) wurde durch Transformation in *E. coli* übertragen.

### c) Konjugationstransfer von Cosmiden oder Plasmiden in B. glumae

Die Konjugationen der Cosmide erfolgten mit *E.coli* JM101 als Donor und *E*. *coli* pRK2013 als Helferstamm [6]. Konjugationen mit den Plasmiden mit breiter Wirtsspezifität pBBR1mcs und pBBRpro (hergestellt aus pBBR1mcs durch Einklonieren des pro-Gens über die Schnittstellen *Hind*III und *Bam*HI) wurden unter Verwendung von *E. coli* S17-1 als Donorstamm biparental durchgeführt [19]. Über-Nacht-Kulturen der Rezipienten (*B. glumae* PG1 oder LU8093) sowie logarithmische Kulturen der Donor- und Helferstämme wurden zentrifugiert, gewaschen und in 0,9 % NaCl resuspendiert. 1-ml-Fraktionen jeder Kultur wurden kombiniert, zentrifugiert und in 50 µl 0,9 % NaCl resuspendiert. Die Mischungen wurden auf LB-Agarplatten aufgetropft und getrocknet. Die Platten wurden über Nacht bei 30 °C inkubiert. Am nächsten Tag wurden die Spots in 0,9 % NaCl resuspendiert und auf selektivem PG-Medium, enthaltend Tetracyclin (50 µg/ml) und Ampicillin (100 µm/ml) für das Cosmid-Screening oder enthaltend Chloramphenicol (200 µg/ml) und Ampicillin (100 µg/ml) für die Selektion der Vektoren mit breiter Wirtsspezifität ausplattiert.

### d) Expressionsuntersuchungen im homologen Wirt B. glumae und Überexpression des rekombinanten Proteins im heterologen Wirt E. coli

Die Expression von pBBRpro in den *B. glumae*-Stämmen PG1 und Lu8093 erfolgte über 24 Stunden unter Verwendung von PG-Medium + 1 % Olivenöl. Als Kontrolle wurde der leere Vektor pBBR1mcs exprimiert. Nach 24 Stunden wurden Proben genommen, zentrifugiert und zweimal mit 0,9 % NaCl gewaschen. Geeignete Verdünnungen der Pellets wurden zur Bestimmung der optischen Dichte (OD₅₈₀) verwendet, während der Überstand zur Bestimmung der Lipaseaktivität eingesetzt wurde.

Die Überexpression der erfindungsgemäßen Protease wurde in *E. coli* durchgeführt, wobei der T7-Expressionswirt *E. coli* BL21 DE3 verwendet wurde. Der Stamm wurde in LB-Medium, enthaltend 100 µg/ml Ampicillin für die Erhaltung des überexprimierenden Vektors pETpro gezüchtet. Transformation des Vektors pETpro in *E.coli* BL21 DE3 erfolgte mittels Hitzeschock (nach RbCl₂ Methode von Hanahan, 1983 [32]) durch Zugabe von 1µl Vektor in 50µl TMF-Puffer auf einen Ansatz transformationskompetenter Zellen, Inkubation auf Eis für 30 Minuten, Hitzeschock bei 42°C für 90 Sekunden, Zugabe von 700µl LB-Medium und Inkubation des Ansatzes für 30 Minuten bei 37°C. Anschließend erfolgt ein Ausplattieren des Ansatzes auf Selektivagarplatten (LB-Agar mit Ampicillin 100µg/ml).

Nach 2-stündiger Inkubation bei 37 °C wurde die Expression des T7-Promotors durch Zugabe von IPTG in einer Endkonzentration von 0,4 mM. induziert. Proben der Kultur wurden nach 2, 4 und 6 Stunden genommen und durch Natriumdodecylsulfat (SDS)-Polyacrylamid-Gelelektrophorese analysiert.

### e) Herstellung von Zellextrakten

Die Zellen wurden auf eine optische Dichte von 0,15 eingestellt, in 15 µl SDS-Puffer resuspendiert und 5 Minuten bei 95 °C inkubiert.

### f) TCA-Fällung

1/10 Volumen von 1 %igem Natriumdodecylsulfat wurde zum Überstand zugegeben und 10 Minuten bei Zimmertemperatur inkubiert. Die Proteine wurden mit 1/10 Volumen 70 %iger Trichloressigsäure (TCA) und einstündiger Inkubation auf Eis ausgefällt. Nach 10-minütiger Zentrifugation (13000 Upm, Zimmertemperatur) wurden die Proben zweimal mit eiskaltem 80 %igem Aceton gewaschen und im Vakuumtrockner getrocknet. Schließlich wurde das Protein in 15 µl SDS-Puffer resuspendiert und 5 Minuten bei 95 °C inkubiert.

### g) SDS-Polyacrylamid-Gelelektrophorese

Natriumdodecylsulfat-Polyacrylamid-Gelelektrophorese (SDS-PAGE) wurde mit 15 %-igem Polyacrylamid-Gel nach Laemmli [13] durchgeführt. Die Proteine wurden mit Coomassie Brilliant Blue R250 gefärbt.

### h) Native Polyacrylamid-Gelelektrophorese

Native Polyacrylamid-Gelelektrophorese wurde unter Verwendung eines 4 bis 12 %-igen Polyacrylamid-Gradientengels (Invitrogen) nach Herstellerprotokoll durchgeführt. Die Proteine wurden mit Coomassie Brilliant Blue G250 gefärbt.

### i) Reinigung der erfindungsgemäßen Protease

Die Reinigung der erfindungsgemäßen Protease erfolgte durch Nickel-Nitrilotriessigsäure-Metall-Affinitätschromatographie. Hierzu wurden 6 I Flüssigkultur von *E. coli* BL21 DE3 pETpro bei 37 °C bis zu einer optischen Dichte bei 580 nm von 5,0 gezüchtet. Durch Zugabe von 1 mM IPTG wurden die Zellen anschließend induziert und weitere 4 Stunden bei 37 °C gezüchtet. Nach Ernte der Zellen durch Zentrifugation (5000 Upm, 15 Minuten, 4 °C) wurden die Pellets in 25 ml eisgekühltem Puffer (50 mM Kpi, 10 mM Imidazol, pH 8,0) resuspendiert. Der Zellaufschluss erfolgte durch Inkubation mit Lysozym (0,5 mg/ml, 30 Minuten auf Eis), gefolgt durch Beschallung (10 x 30 Sekunden). Um Zellfragmente zu entfernen, wurde die Probe erneut zentrifugiert (14000 Upm, 20 Minuten, 4 °C) und filtriert. Das klare Lysat wurde auf eine Nickel-Nitrilotriessigsäure-Säule aufgetragen und mit Kaliumphosphatpuffer (50 mM Kpi, 20 mM Imidazol, pH 8,0) gewaschen. Nach Elution des rekombinanten Proteins mit 50 mM Kaliumphosphatpuffer, enthaltend 250 mM Imidazol (pH 8,0) wurden die entsprechenden Fraktionen vereinigt und unter Verwendung einer Sephadex-G25-Säule entsalzt. Ein Volumen von 50 µl eines jeden Reinigungsschritts wurde für die SDS-PAGE-Analyse und die proteolytische Enzymaktivitätsmessung zurückgestellt. Die Proteinkonzentrationen wurden anschließend unter Verwendung eines Bradford-Assays [4] und Verwendung von Rinderserumalbumin als Standard bestimmt.

### j) Enzymaktivitäts-Assays

Die lipolytische Aktivität wurde auf Indikatorplatten bestimmt, die eine Tributyrin-Emulsion (7,5 ml Tributyrin, 0,75 g Gummi arabicum, ad 15 ml destilliertes Wasser) enthielten oder alternativ spektrophotometrisch mit p-Nitrophenylpalmitat (pNPP) als Substrat, wie bei Stuer at al. [21] beschrieben.
pNPP: 0,8 mM Endkonzentration
Sorensenphosphatpuffer: Lösung A: 50 mM Na₂HPO₄ x 2 H₂O
Lösung B: 50 mM KH₂PO₄
Lösungen A und B werden im Verhältnis 17:1 gemischt
Substratemulsion: Lösung I: 207 mg Natriumdeoxycholat
100 mg Gummi arabicum
90 ml Sørensenphosphatpuffer
Lösung II: 30 mg p-Nitrophenylpatmitat
10 ml Isopropanol

Die Substratemulsion wird vor jeder Aktivitätsbestimmung frisch angesetzt und innerhalb einer Stunde verbraucht. Der Enzymtest wird konstant bei 37° durchgeführt. Dabei werden 10-50 µl eines Überstandes zu 2 ml vorgewärmter Substratemulsion zugesetzt und die Veränderung der OD₄₁₀ (ΔOD₄₁₀) über einen Zeitraum von 5 Minuten gemessen. Die relative lipolytische Aktivität ergibt sich durch die Korrelation der ΔOD₄₁₀/min mit der OD₅₈₀ der Kulturen.

Die proteolytische Aktivität wurde unter Verwendung von Magermilch-Agarplatten bestimmt, welche pro Liter enthielten: 30 g Magermilchpulver, 5 g Hefeextrakt, 10 g NaCl, 10 g Trypton, 15 g Agar und 1 mM IPTG. Einzelne Kolonien der Expressionskulturen oder 5 µl eines jeden Reinigungsschrittes wurden auf Agarplatten aufgetragen und über Nacht bei 30 °C *(B. glumae)* oder 37 °C *(E. coli)* inkubiert. Die proteolytische Aktivität kann durch Bildung von Höfen um die Kolonien oder Proben herum nachgewiesen werden.

### k) Computeranalysen

Die Sequenzalignments, orf und Datenbankrecherchen wurden unter Verwendung von Standardprogrammen des National Center for Biotechnology Information: Basic Local Alignment Search Tool (BLAST) [1, 2], Open Reading Frame-Finder (ORF-Finder) oder European Bioinformatics Institute (EMBL-EBI): Washington University Basic Local Alignment Search Tool Version 2.0 (WU-BLAST2, db Clustal) [1,210] durchgeführt. Die Vorhersage einer möglichen Lokalisation des Proteins und Computeranalyse wurden unter Verwendung der Vorhersageprogramme, ProtParam tool [9], PSORT-B [8], SignalP 3.0 Server [14] des Expert Protein Analysis Systems (ExPASY) und des Helix-Tum-Helix-Vorhersage-Servers der Pole Biolriforrnatic, Lyon, Network Protein Sequence Analysis (PBIL, NPS, France) [5] durchgeführt.

### 2. Versuchsergebnisse

### a) Identifikation von Engpässen der Lipaseproduktion von B. glumae

Eine genomische Bibliothek von *B. glumae* PG1 wurde, wie oben beschrieben, unter Verwendung des Cosmids pLAFR3 [19] mit breiter Wirtsspezifität konstruiert. Nach Konjugation in *B. glumae* PG1 und LU8093 wurde die Cosmid-Bank unter Verwendung von Lipaseindikatorplatten und Lipaseaktivitätsassays [21] gescreent.

In einer Bibliothek von etwa 2500 Klonen wurden 15 Cosmide identifiziert, welche die Lipaseproduktion beeinflussten. Die entsprechenden DNA-Fragmente aus zweien dieser Cosmide wurden in pBBR1 mcs subkloniert und durch DNA-Sequenzierung identifiziert. Weitere Expressionsuntersuchungen in *B. glumae* ergaben schließlich ein Plasmid, das zu einer 20 bis 30 %igen Zunahme der extrazellulären Lipaseaktivität führte (vgl. Figur 3).

### b) Expression eines ThiJ/Pfpl-Proteins führt zu einer erhöhten Lipaseaktivität in in B. glumae

Die Restriktionsanalyse des Plasmids pBBRPG 8/1 (Figur 3) und die DNA-Sequenzierung des Inserts ergaben eine DNA-Fragmentgröße von etwa 1,2 kb (vgl. Figur.4, Bahn 4).

Unter Verwendung des Open Reading Frame-Suchprogramms (ORF-Finder) des National Center for Biotechnology Information (NCBI) wurden drei offene Leserahmen (orfs) detektiert, wovon der größte (540 bp) für ein Protein mit einer konservierten Domäne kodiert, welche für Proteine der ThiiJ/Pfpl-Familie charakteristisch ist. Die anderen zwei orfs umfassen 444 bzw. 348 bp und zeigten keine signifikante Ähnlichkeit mit anderen annotierten Proteinen der Datenbank.

Ein Vergleich der Aminosäuresequenzen mit anderen Proteinen in der Datenbank unter Verwendung des Programms WU-Blast2 von EMBL-EBI ergab signifikante Ähnlichkeiten in mehreren Fällen, insbesondere mit putativen intrazellulären Proteasen und Stressproteinen (vgl. Figur 1). Beispielsweise sind 69 % der Aminosäuresequenz ähnlich mit der intrazellulären Protease YHBO aus *E. coli* [15]. Eine 49 %-ige Ähnlichkeit bestand mit dem allgemeinen Stressprotein YFKM aus *Bacillus clausii* [22], eine 44 %-ige Ähnlichkeit mit Protease I aus *Bacillus cereus* [11] und eine 44 %-ige Übereinstimmung mit der Protease Pfpl und der Protease PH1704 der thermophilen Bakterien *Pyrococcus furiosus* und *P. horikoshii* [25]. Ein kürzlich veröffentlichter Überblick über die evolutionären und funktionalen Zusammenhänge innerhalb der DJ-I/ThiJ/PfpI-Superfamilie zeigt, dass diese Superfamilie Proteine mit unterschiedlichen Funktionen umfasst, wie Proteasen, Transkriptionsregulatoren, Sigma-kreuzreagierende Proteine, Katalasen und dergleichen [3].

Neben dem humanen DJ-1-Protein, das an der vererbten Parkinson-Krankheit beteiligt ist und den ThiJ oder Protease-verwandten Proteinen aus Pflanzen gibt es auch eine Anzahl bakterieller Proteine, welche eine DJ-1/ThiJ-ähnliche Domäne aufweisen, wie beispielsweise die Large-Subunit-Katalasen, enthaltend eine Katalase-Domäne und eine DJ-1/ThiJ-Domäne, oder die Transkriptionsregulatoren vom AraC-Typus. Die Letztgenannten können über die Präsenz einer oder mehrerer Helix-Turn-Helix (HTH)-Motive im C-terminalen Teil des Proteins identifiziert werden. Das HTH-Motiv vermittelt vermutlich die DNA-Bindung, während die ThiJ-ähnliche Domäne eine Amidase ist [3]. Eine außergewöhnliche konservierte Zusammensetzung (91,4 % Identität) zeigt die Familie der Sigma-kreuzreagierenden Proteine, welche diese von den benachbarten Familien unterscheidet. Diese Proteine haben eine distinkte ElbB-Domäne (ElbB = Enhancing lycopene biosynthesis protein 2 aus *E. coli*) und zeigen eine relativ moderate Homologie mit den ThiJ-Proteinen. Schließlich gibt es zwei Gruppen, welche hohe Ähnlichkeit auf der Aminosäuresequenz-Ebene besitzen, zu welchen die Proteine des obigen Alignments (Figur 1) zählen. Die erste Gruppe umfasst die Pfpl-Proteasen und beinhaltet die beiden Proteasen PfPI und PH1704 aus den thermophilen Bakterien *Pyrococcus furiosus bzw. Pyrococcus horikoshii.* Diese Proteasen bilden hexamere Ringstrukturen und zeigen eine ATP-unabhängige Proteaseaktivität, jedoch nur in oligomerer Form. Aufgrund des Vorliegens eines Cystein-Restes (100) in Nachbarstellung zu einem Histidin-Rest werden diese Proteine als Cystein-Proteasen klassifiziert. In der Kristallstruktur von PH1704 ist der entsprechende Cys100-Rest in einem nukleophilen Ellenbogenmotiv angeordnet und bildet zusammen mit His101 den Teil einer katalytischen Triade an der Grenzfläche zwischen drei Paaren von Monomeren [23]. Da all die bekannten Proteasen benachbarte Cys/His-Reste aufweisen, wohingegen eine Substitution durch Cys/X in Nicht-Protease-Mitgliedern gefunden wird, ist die Wahrscheinlichkeit gross, dass viele Proteine der benachbarten Gruppe, nämlich die der ThiJ/Pfpl-ähnlichen Proteine, ebenfalls Proteaseaktivität zeigen, da die meisten davon ein Cys/His-Paar an derselben Position aufweisen. Außerdem konnte eine Konsensus-Sequenz um das Cys/His-Paar, nämlich AICHGP (SEQ ID NO: 7) identifiziert werden. Proteine, welche zu dieser Gruppe zählen, umfassen beispielsweise die intrazelluläre Protease YhbO aus *E. coli,* das Stressprotein GSP18 aus *B. subtilis,* das Chaperon Hsp31 aus *E. coli* und die intrazelluläre Protease YDR533C aus *S. cerevisiae* [3].

Um die erfindungsgemäße Protease in einen der oben beschriebenen Cluster einzuordnen, wurden computergestützte Untersuchungen mit Hilfe der ExPASy-Website durchgeführt. Es wurde ein Molekulargewicht von etwa 19,6 kDa und ein theoretischer pl von 5,45 (ProtParam Tool [9]) bestimmt. Nach dem SignalP 3.0 Vorhersageprogramm [14] umfasst die Aminosäuresequenz keine Signalsequenz für die Sekretion. Dies weist auf eine cytoplasmatische Lokalisierung hin, welche durch das Vorhersageprogramm für die subzelluläre Lokalisation bakterieller Proteine (PSORT-B[8]) bestätigt werden konnte. Ein weiterer struktureller Vergleich unter Verwendung des HTH-Vorhersageprogramms (Network Protein Sequence Analysis [5]) ergab, dass die erfindungsgemäße Protease keine HTH-DNA-Bindungsdomänen aufweist. Folglich scheint das Protein kein transkriptioneller Regulator zu sein

Aufgrund der großen Ähnlichkeit auf Aminosäuresequenzebene mit der intrazellulären Protease YhbO aus *E. coli* ist es wahrscheinlicher, dass die erfindungsgemäße Protease aus *B. glumae* eine hohe Ähnlichkeit mit YhbO besitzt und somit zur Familie der ThiJ/Pfpl-ähnlichen Proteine zählt. Wie dem Alignment (Figur 1) zu entnehmen ist, sind alle konservierten Aminosäurenreste, welche in YhbO und anderen Proteinen dieser Gruppe identifiziert werden konnten, auch in der erfindungsgemäßen Protease zu finden. Die Kristallstruktur von YhbO wurde ebenfalls bestimmt und zeigte, dass das Protein ein Homodimer bildet [25]. Unter Verwendung der nativen Polyacrylamid-Gelelektrophorese konnte gezeigt werden, dass die Untereinheit der erfindungsgemäßen Protease, welche als Fusionsprotein in *E. coli* exprimiert wird, eine multimere Konformation ausbildet (Daten nicht gezeigt).

### c) Expression der erfindungsgemäßen Protease in B. glumae

Die Analyse der DNA-Sequenz erlaubte die Bereitstellung zweier Primer zur Amplifizierung des Gens der erfindungsgemäßen Protease direkt aus dem Genom *von B. glumae* PG1. Das resultierende PCR-Produkt wurde in den Vektor pBBR1mcs mit breiter Wirtsspezifität unter Verwendung der Restriktionsstellen HindIII und BamHI insertiert. Das Konstrukt mit der Bezeichnung pBBRpro wurde unter Verwendung von *E. coli* S17-1 als Donorstamm und *B. glumae* PG1 bzw. LU8093 als Rezipienten transferiert. Insgesamt wurden vier verschiedene Expressionsstudien wie oben beschrieben durchgeführt.

Im Falle des Wildtypstamms *B. glumae* PG1 führte die zusätzliche Expression des Plasmids pBBRpro zu einer 2- bis 3-fachen Zunahme der Lipaseaktivität, während im Produktionsstamm LU8093 eine 0,3-fache Zunahme zu beobachten war (vgl. Figuren5a und b). Der Grund für diesen Unterschied kann darin zu sehen sein, dass der Produktionsstamm bereits eine etwa 10-fach erhöhte Lipasemenge als der Wildtyp produziert, so dass die Expression des Plasmids die Lipaseproduktion im gleichen Ausmaß, wie im Wildtypstamm beobachtet, nicht mehr bewirken kann.

Werden Kolonien aus den Expressionskulturen auf Magermilch-Agarplatten gezüchtet, beobachtet man keinerlei Unterschiede in der Hof-Bildung (Daten nicht gezeigt). Da die erfindungsgemäße Protease vermutlich im Cytoplasma lokalisiert ist und der Vektor pBBR1mcs eine relativ niedrige Kopienzahl aufweist, sind einfache Plattenkulturen auf den Indikatorplatten möglicherweise nicht ausreichend, um proteolytische Aktivität nachzuweisen.

### d) Überexpression der erfindungsgemäßen Protease in E. coli

Für die Überexpression der erfindungsgemäßen Protease wurde das Gen in den Vektor pET22b unter der Kontrolle des Promotors T7 kloniert und das resultierende Konstrukt wurde in *E. coli* BL21 DE3 transformiert. Erste Überexpressionsuntersuchungen wurden in kleinem Maßstab mit 25-ml-Kulturen, wie oben beschrieben, durchgeführt und Proben wurden durch SDS-PAGE analysiert (vgl. Figur 6). Eine erfolgreiche Überexpression des Proteins konnte 2 bis 6 Stunden nach der Induktion nachgewiesen werden. Ausgehend vom verwendeten Proteinstandard hat die erfindungsgemäße Protease ein Molekulargewicht von etwa 21 kDa, was geringfügig über dem berechneten Molekulargewicht von etwa 19,6 kDa liegt. Wenn Kolonien, die aus Expressionskulturen gewonnen wurden, auf Magermilch-Agarplatten gezüchtet werden, beobachtet man keine Unterschiede in der Hof-Bildung (Daten nicht gezeigt). Da man zeigen konnte, dass die Überexpression der erfindungsgemäßen Protease nicht zur Bildung von Einschlusskörpern führt, wird angenommen, dass das Protein bei Expression in *E. coli* inaktiv ist, oder dass Magermilch kein geeignetes Substrat darstellt.

### e) Reinigung der erfindungsgemäßen Protease

Das überexprimierte Protein wurde mit Hilfe der Nickel-Nitrilotriessigsäure-Metall-Affinitätschromatographie gereinigt. 6-Liter-Kulturen von *E. coli* BL21 DE3 pETpro wurden über Nacht bei 37 °C bis zu einer optischen Dichte OD₅₈₀ von 0,6 gezüchtet und die Überexpression des Proteins wurde durch Zugabe von 1 mM IPTG induziert. Nach 4 Stunden wurden die Zellen geerntet und in 25 ml eiskaltem Puffer (50 mM Kpi, 10 mM Imidazol, pH 8,0) resuspendiert. Die Zerstörung der Zellen erfolgte durch Inkubation mit Lysozym (0,5 mg/ml, 30 Minuten auf Eis) gefolgt von einer Beschallung (10 x 30 Sekunden). Um Zellfragmente zu entfernen, wurden die Proben zentrifugiert (14000 Upm, 20 Minuten, 4 °C) und filtriert. Das klare Lysat wurde anschließend auf eine Nickel-Acetonitril-Säule aufgetragen und mit Kaliumphosphatpuffer (50 mM Kpi, 10 mM Imidazol, pH 8,0) gewaschen. Nach Elution des rekombinanten Proteins mit 50 mM Kpi-Puffer, enthaltend 250 mM Imidazol (pH 8,0) wurden die entsprechenden Fraktionen vereinigt und unter Verwendung einer Sephadex- G25-Säule entsalzt. Ein Volumen von 50 µl wurde für jeden Reinigungsschritt für die SDS-PAGE-Analyse (Figur 7) entnommen. Nach Entsalzen des Eluats betrug das Gesamtvolumen des gereinigten Proteins 90 ml. Nach Brädford-Assay unter Verwendung von Rinderserumalbumin als Standard wurde eine Proteinkonzentration der Probe von 3 mg/ml bestimmt, was einer Gesamtausbeute an Protein von 270 mg entsprach. Dieses gereinigte Protein kann nun weiteren Enzymaktivitäts-Tests unterzogen werden.

### f) Bestimmung der Protease-Aktivität im Mikrotiterplattentest

Das gereinigte Protein wurde in einem Enzymplattentest (Taxaprofile E, Merlin, Bornheim-Hersel) auf Proteaseaktivität getestet. Bei diesem Test wird das zu untersuchende Protein in Substraten für 95 Aminopeptidasen und Proteasen, 76 Glucosidasen, Phosphatasen und Esterasen sowie in 17 klassischen Reaktionen bei pH8,2, pH7,5, pH5,5 und pH4,0 getestet. Die Durchführung erfolgte nach dem von der Firma Merlin mitgelieferten Protokoll. Also Kontrolle diente der Puffer, in dem das gereinigte Protein vorlag (10 mM Kpi Puffer pH6,5). Nach einer Inkubation der Platten für 24 h bei 30°C erfolgte die Auswertung visuell. Insgesamt wurde dieser Versuch dreimal durchgeführt, wobei eindeutig positive Reaktionen bei den folgenden Substraten festgestellt werden konnten:
Vier Aminopeptidase-Substrate: Lysin-β-Na, Arginin-β-Na, L-Alanin-β-Na, Glutamat(βNa)-OH
Zwei Glucosidase-Substrate: p-Nitrophenyl-alpha-L-arabinopyranosid, p-Nitrophehyl-β-D-galactopyranosid.

### Literaturverzeichnis

[1] Altschul S.F., Gish W., Miller W., Myers E.W. & Lipman D.J. (1990) Basic local alignment search tool, J. Mol. Biol. 215: 403-410
[2] Altschul S.F., Madden T.L., Schaffer A. A., Zhang J., Zhang Z., Miller W. & Lipman D.J. (1997) Gapped BLAST and PSI-Blast: a new generation of protein database search programs. Nucleic Acids Res. 25 (17): 3389-402
[3] Bandyopadhyay S., Cookson M.R. (2004) Evolutionary and functional relationships within the DJ1 superfamily. BMC Evol Biol 4 (6):1-9
[4] Bradford M. (1976): A rapid and sensitive method for the quantitation of microgram quantities of protein utilizing the principles of protein-dye binding. Anal. Biochem. 72: 248-254
[5] Dodd I.B., Egan J.B. (1990) Helix-turn-helix DNA-binding motifs prediction: Improved detection of helix-turn-helix DNA-binding motifs in protein sequences. Nucleic Acids Res 18 (17):5019-5026
[6] Figurski D.H. & Helinski D. R. (1979), Replication of an origin-containing derivative of plasmid RK2 dependent on a plasmid function provided in trans. Proc. Natl. Acad. Sci USA 76 (4): 1648-1652
[7] Frenken, L. (1993) Characterization, biogenesis and protein engineering. PhD Thesis, University of Utrecht[8] Gardy J.L., Spencer C., Wang K., Ester M., Tusnady G.E., Simon I., Hua S., deFays K., Lambert C., Nakai K. & Brinkman F.S.L. (2003) PSORT-B: improving protein subcellular localization prediction for Gram-negative bacteria. Nucleic Acids Research 3 (13): 3613-17
[9] Gill S.C., von Hippel P.H. (1989) Calculation of protein extinction coefficients from amino acid sequence data. Anal. Biochem. 182: 319-326
[10] Higgins D., Thompson J., Gibson T.Thompson J.D., Higgins D.G., Gibson T.J. (1994), CLUSTAL W: improving the sensitivity of progressivemultiple sequence alignment through sequence weighting,position-specific gap penalties and weight matrix choice. Nucleic Acids Res. 22: 4673-4680
[11] Ivanova N., Sorokin A., Anderson I., Galleron N., Candelon B., Kapatral V., Bhattacharyya A., Reznik G., Mikhailova N., Lapidus A., Chu L., Mazur M., Goltsman E., Larsen N., D'Souza M., Walunas T., Grechkin Y., Pusch G., Haselkorn R., Fonstein M., Ehrlich S.D., Overbeek R., Kyrpides N.C. (2003) Genome sequence of Bacillus cereus and comparative analysis with Bacillus anthracis. Nature 423:87-91
[12] Kunst F., et al (1997) The complete genome sequence of the Gram-positive bacterium Bacillus subtilis. Nature 390: 249-256
[13] Laemmli U.K. (1970) Cleavage of structural proteins during the assembly of the head of bacteriophage T4, Nature 227 (259): 680-5
[14] Nielsen H., Engelbrecht J., Brunak S. & von Heijne G. (1997) Identification of prokaryotic and eukaryotic signal peptides and prediction of their cleavage sites. ProteinEngineering 10:1-6
[15] Pema N.T., Plunkett G. III, Burland V., Mau B. (2001) Genome sequence of enterohaemorrhagic Escherichia coli O157:H7. Nature 409:529-533
[16] Rao M.B., Tanksale A.M., Ghatge M.S., Deshpande V.V. (1998) Molecular and biotechnological aspects of micobial proteases. Microbiol. Mol. Biol. Rev. 62: 597-635
[17] Sambrook J., Fritsch E.F., Maniatis T. (1989) Molecular cloning: a laboratory manual, 2nd ed. Cold Spring Harbor Laboratory Press, Cold Spring Harbor, N.Y.
[18] Schmid A., Dordick J.S., Hauer B., Kiener A., Wubbolts M. & Witholt B. (2001) Industrial biocatalysis today and tomorrow. Nature insight 409: 258-268
[19] Simon R., Reifer U. & Puehler A. (1983) A broad host range mobilization system for in vivo genetic engineering: Transposon mutagenesis in Gram-negative bacteria. BioTechnology 1: 784-791
[20] Staskawicz B., Dahlbeck D., Keen N. & Napoli C. (1987) Molecular characterization of cloned avirulence genes form race 0 and race 1 of Pseudomonas syringae pv. Glycinea. J. Bacteriol, 169: 5789-5794
[21] Stuer W., Jaeger K.E. & Wickler U.K. (1986) Purification of extracellular lipase from Pseudomonas aeruginosa. J. Bacteriol. 168: 1070-1074
[22] Takaki Y., Kageyama Y., Shimamura S., Suzuki H., Nishi S. (2003) The complete genome sequence of the alkaliphilic Bacillus clausii KSM-K16. Submitted to the EMBUGenBank/DDBJ databases
[23] Trotter E.W., Kao. C.M., Berenfeld L., Botstein D., Petsko G.A., & Gray J.V. (2002) Misfolded proteins are competent to mediate a subset of the responses to heat shock in Saccharomyces cerevisiae. J. Biol. Chem. 277: 44817-44825
[24] Windgassen M. (2000) Proteinsekretion in Pseudomonas aeruginosa: Untersuchung der Rolle periplasmatischer Proteasen durch Insertionsmutagenese. Diplomarbeit, Ruhr-Universität Bochum
[25] Xinlin D., In-Geol C., Rosalind W., Weiru W., Jaru J., Hisao Y., Sung-Hou K. (2000) Crystal structure of an intracellular protease from Pyrococcus horikoshii at 2-Å resolution. PNAS 97 (26): 14079-14084
[26] Dodd I.B., Egan J.B. (1990) Helix-turn-helix DNA-binding motifs prediction: Improved detection of helix-turn-helix DNA-binding motifs in protein sequence. Nucleic Acids Res 18 (17): 5019-5026
[27] Breuer, M., Ditrich, K., Habicher, T., Hauer, B., Keßeler, M., Stürmer, R. and Zelinski, T. (2004) Industrial methods for the production of optically active intermediates. Angew. Chem. Int. Ed. 43: 788-824
[28] Jaeger, K.-E.& Eggert, T. (2002) Lipases for biotechnology. Curr. Opin. Biotechnol. 13: 390-397
[29] Schmid A., Dordick J.S., Hauer B., Kiener A., Wubbolts M. & Witholt B. (2001) Industrial biocatalysis today and tomorrow. Nature insight 409: 258-268
[30] Kovach, M.E. et al. Phillips, R.W., Elzer, P.H., Roop, R.M. and Peterson, K.M. (1994) pBBR1MCS: a broad-host-range cloning vector. BioTechniques 16 (5), 800-802
[31] Winkler, U.K. & Stuckmann, M.: Glycogen, hyaloronate, and some other polysaccharides greatly enhance the formation of exolipase by Serratiamarcescens. J. Bacteriol. 1978; 138: 663-670
[32] Hanahan D.: Studies on transformation of Escherichia coli with plasmids. J Mol Biol. 1983; 166:557-80

### SEQUENCE LISTING

<110> BASF Aktiengesellschaft
<120> Verfahren zur Herstellung von Lipasen
<130> M/46033
<160> 11
<170> PatentIn version 3.1
<210> 1
   <211> 540
   <212> DNA
   <213> Burkholderia glumae
<400> 1
   atgcagcagc gtggcggcag catgagcaag aagatcgccg tcctggccgt ggacgaattc      60
   gaagacagcg aactggtcga acccctgcgg gcgctgcgta aggccggcgc cgaagtcgac     120
   gtgatcagcc agcaggcggg cgaagtcaag gggttccgtc acgcggacaa gggggaagcc     180
   gtcaaggtgg accgtacgtt cgacgaagtc cgggaaggcg aattcgacgc gctgctgctg     240
   cccggcggcc tgctgcgtgg cgacaatcgg atgctgcccg cggcccgtga attcgtgacc     300
   gccggcaagc cggtcttcgc gatctgccac ggcgggtggc tgctgggcag cagcggggtc     360
   atcaacggcc gcaagctgac cgcctggccc agcctgcagg acgacgtgaa gaatgcgggc     420
   ggcgaattct acgaccagga agtggtgcgt gacaaggacc agctgatcac cagccgtaag     480
   cccgacgacc tgccggcctt caaccgggaa gcgctgcggc tgctgggggc cggcgcgtga     540
<210> 2
   <211> 179
   <212> PRT
   <213> Hurkholderia glumae
<400> 2
   Met Gln Gln Arg Gly Gly Ser Met Ser Lys Lys Ile Ala Val Leu Ala
   1               5                   10                  15
   Val Asp Glu Phe Glu Asp Ser Glu Leu Val Glu Pro Leu Arg Ala Leu
               20                  25                  30
   Arg Lys Ala Gly Ala Glu Val Asp Val Ile Ser Gln Gln Ala Gly Glu
           35                  40                  45
   Val Lys Gly Phe Arg His Ala Asp Lys Gly Glu Ala Val Lys Val Asp
       50                  55                  60
   Arg Thr Phe Asp Glu Val Arg Glu Gly Glu Phe Asp Ala Leu Leu Leu
   65                  70                  75                  80
   Pro Gly Gly Leu Leu Arg Gly Asp Asn Arg Met Leu Pro Ala Ala Arg
                   85                  90                  95
   Glu Phe Val Thr Ala Gly Lys Pro Val Phe Ala Ile Cys His Gly Gly
               100                 105                 110
   Trp Leu Leu Gly Ser Ser Gly Val Ile Asn Gly Arg Lys Leu Thr Ala
           115                 120                 125
   Trp Pro Ser Leu Gln Asp Asp Val Lys Asn Ala Gly Gly Glu Phe Tyr
       130                 135                 140
   Asp Gln Glu Val Val Arg Asp Lys Asp Gln Leu Ile Thr Ser Arg Lys
   145                 150                 155                 160
   Pro Asp Asp Leu Pro Ala Phe Asn Arg Glu Ala Leu Arg Leu Leu Gly
                   165                 170                 175
   Ala Gly Ala
<210> 3
   <211> 5238
   <212> DNA
   <213> Artificial Sequence
<220>
<223> Plasmid
<220>
<221> misc-feature
<223> Plasmid
<400> 3
   ctcgggccgt ctcttgggct tgatcggcct tcttgcgcat ctcacgcgct cctgcggcgg 60
   cctgtagggc aggctcatac ccctgccgaa ccgcttttgt cagccggtcg gccacggctt 120
   ccggcgtctc aacgcgcttt gagattccca gcttttcggc caatccctgc ggtgcatagg 180
   cgcgtggctc gaccgcttgc gggctgatgg tgacgtggcc cactggtggc cgctccaggg 240
   cctcgtagaa cgcctgaatg cgcgtgtgac gtgccttgct gccctcgatg ccccgttgca 300
   gccctagatc ggccacagcg gccgcaaacg tggtctggtc gcgggtcatc tgcgctttgt 360
   tgccgatgaa ctccttggcc gacagcctgc cgtcctgcgt cagcggcacc acgaacgcgg 420
   tcatgtgcgg gctggtttcg tcacggtgga tgctggccgt cacgatgcga tccgccccgt 480
   acttgtccgc cagccacttg tgcgccttct cgaagaacgc cgcctgctgt tcttggctgg 540
   ccgacttcca ccattccggg ctggccgtca tgacgtactc gaccgccaac acagcgtcct 600
   tgcgccgctt ctctggcagc aactcgcgca gtcggcccat cgcttcatcg gtgctgctgg 660
   ccgcccagtg ctcgttctct ggcgtcctgc tggcgtcagc gttgggcgtc tcgcgctcgc 720
   ggtaggcgtg cttgagactg gccgccacgt tgcccatttt cgccagcttc ttgcatcgca 780
   tgatcgcgta tgccgccatg cctgcccctc ccttttggtg tccaaccggc tcgacggggg 840
   cagcgcaagg cggtgcctcc ggcgggccac tcaatgcttg agtatactca ctagactttg 900
   cttcgcaaag tcgtgaccgc ctacggcggc tgcggcgccc tacgggcttg ctctccgggc 960
   ttcgccctgc gcggtcgctg cgctcccttg ccagcccgtg gatatgtgga cgatggccgc 1020
   gagcggccac cggctggctc gcttcgctcg gcccgtggac aaccctgctg gacaagctga 1080
   tggacaggct gcgcctgccc acgagcttga ccacagggat tgcccaccgg ctacccagcc 1140
   ttcgaccaca tacccaccgg ctccaactgc gcggcctgcg gccttgcccc atcaattttt 1200
   ttaattttct ctggggaaaa gcctccggcc tgcggcctgc gcgcttcgct tgccggttgg 1260
   acaccaagtg gaaggcgggt caaggctcgc gcagcgaccg cgcagcggct tggccttgac 1320
   gcgcctggaa cgacccaagc ctatgcgagt gggggcagtc gaaggcgaag cccgcccgcc 1380
   tgccccccga gcctcacggc ggcgagtgcg ggggttccaa gggggcagcg ccaccttggg 1440
   caaggccgaa ggccgcgcag tcgatcaaca agccccggag gggccacttt ttgccggagg 1500
   gggagccgcg ccgaaggcgt gggggaaccc cgcaggggtg cccttctttg ggcaccaaag 1560
   aactagatat agggcgaaat gcgaaagact taaaaatcaa caacttaaaa aaggggggta 1620
   cgcaacagct cattgcggca ccccccgcaa tagctcattg cgtaggttaa agaaaatctg 1680
   taattgactg ccacttttac gcaacgcata attgttgtcg cgctgccgaa aagttgcagc 1740
   tgattgcgca tggtgccgca accgtgcggc accctaccgc atggagataa gcatggccac 1800
   gcagtccaga gaaatcggca ttcaagccaa gaacaagccc ggtcactggg tgcaaacgga 1860
   acgcaaagcg catgaggcgt gggccgggct tattgcgagg aaacccacgg cggcaatgct 1920
   gctgcatcac ctcgtggcgc agatgggcca ccagaacgcc gtggtggtca gccagaagac 1980
   actttccaag ctcatcggac gttctttgcg gacggtccaa tacgcagtca aggacttggt 2040
   ggccgagcgc tggatctccg tcgtgaagct caacggcccc ggcaccgtgt cggcctacgt 2100
   ggtcaatgac cgcgtggcgt ggggccagcc ccgcgaccag ttgcgcctgt cggtgttcag 2160
   tgccgccgtg gtggttgatc acgacgacca ggacgaatcg ctgttggggc atggcgacct 2220
   gcgccgcatc ccgaccctgt atccgggcga gcagcaacta ccgaccggcc ccggcgagga 2280
   gccgcccagc cagcccggca ttccgggcat ggaaccagac ctgccagcct tgaccgaaac 2340
   ggaggaatgg gaacggcgcg ggcagcagcg cctgccgatg cccgatgagc cgtgttttct 2400
   ggacgatggc gagccgttgg agccgccgac acgggtcacg ctgccgcgcc ggtagcactt 2460
   gggttgcgca gcaacccgta agtgcgctgt tccagactat cggctgtagc cgcctcgccg 2520
   ccctatacct tgtctgcctc cccgcgttgc gtcgcggtgc atggagccgg gccacctcga 2580
   cctgaatgga agccggcggc acctcgctaa cggattcacc gtttttatca ggctctggga 2640
   ggcagaataa atgatcatat cgtcaattat tacctccacg gggagagcct gagcaaactg 2700
   gcctcaggca tttgagaagc acacggtcac actgcttccg gtagtcaata aaccggtaaa 2760
   ccagcaatag acataagcgg ctatttaacg accctgccct gaaccgacga ccgggtcgaa 2820
   tttgctttcg aatttctgcc attcatccgc ttattatcac ttattcaggc gtagcaccag 2880
   gcgtttaagg gcaccaataa ctgccttaaa aaaattacgc cccgccctgc cactcatcgc 2940
   agtcggccta ttggttaaaa aatgagctga tttaacaaaa atttaacgcg aattttaaca 3000
   aaatattaac gcttacaatt tccattcgcc attcaggctg cgcaactgtt gggaagggcg 3060
   atcggtgcgg gcctcttcgc tattacgcca gctggcgaaa gggggatgtg ctgcaaggcg 3120
   attaagttgg gtaacgccag ggttttccca gtcacgacgt tgtaaaacga cggccagtga 3180
   gcgcgcgtaa tacgactcac tatagggcga attggagctc caccgcggtg gcggccgctc 3240
   tagaactagt ggatccaatt cgcctcatcc aatgcagcag cgtggcggca gcatgagcaa 3300
   gaagatcgcc gtcctggccg tggacgaatt cgaagacagc gaactggtcg aacccctgcg 3360
   ggcgctgcgt aaggccggcg ccgaagtcga cgtgatcagc cagcaggcgg gcgaagtcaa 3420
   ggggttccgt cacgcggaca agggggaagc cgtcaaggtg gaccgtacgt tcgacgaagt 3480
   ccgggaaggc gaattcgacg cgctgctgct gcccggcggc ctgctgcgtg gcgacaatcg 3540
   gatgctgccc gcggcccgtg aattcgtgac cgccggcaag ccggtcttcg cgatctgcca 3600
   cggcgggtgg ctgctgggca gcagcggggt catcaacggc cgcaagctga ccgcctggcc 3660
   cagcctgcag gacgacgtga agaatgcggg cggcgaattc tacgaccagg aagtggtgcg 3720
   tgacaaggac cagctgatca ccagccgtaa gcccgacgac ctgccggcct tcaaccggga 3780
   agcgctgcgg ctgctggggg ccggcgcgtg aaagcttatc gataccgtcg acctcgaggg 3840
   ggggcccggt acccagcttt tgttcccttt agtgagggtt aattgcgcgc ttggcgtaat 3900
   catggtcata gctgtttcct gtgtgaaatt gttatccgct cacaattcca cacaacatac 3960
   gagccggaag cataaagtgt aaagcctggg gtgcctaatg agtgagctaa ctcacattaa 4020
   ttgcgttgcg ctcactgccc gctttccagt cgggaaacct gtcgtgccag ctgcattaat 4080
   gaatcggcca acgcgcgggg agaggcggtt tgcgtattgg gcgcatgcat aaaaactgtt 4140
   gtaattcatt aagcattctg ccgacatgga agccatcaca aacggcatga tgaacctgaa 4200
   tcgccagcgg catcagcacc ttgtcgcctt gcgtataata tttgcccatg gtgaaaacgg 4260
   gggcgaagaa gttgtccata ttggccacgt ttaaatcaaa actggtgaaa ctcacccagg 4320
   gattggctga gacgaaaaac atattctcaa taaacccttt agggaaatag gccaggtttt 4380
   caccgtaaca cgccacatct tgcgaatata tgtgtagaaa ctgccggaaa tcgtcgtggt 4440
   attcactcca gagcgatgaa aacgtttcag tttgctcatg gaaaacggtg taacaagggt 4500
   gaacactatc ccatatcacc agctcaccgt ctttcattgc catacggaat tccggatgag 4560
   cattcatcag gcgggcaaga atgtgaataa aggccggata aaacttgtgc ttatttttct 4620
   ttacggtctt taaaaaggcc gtaatatcca gctgaacggt ctggttatag gtacattgag 4680
   caactgactg aaatgcctca aaatgttctt tacgatgcca ttgggatata tcaacggtgg 4740
   tatatccagt gatttttttc tccattttag cttccttagc tcctgaaaat ctcgataact 4800
   caaaaaatac gcccggtagt gatcttattt cattatggtg aaagttggaa cctcttacgt 4860
   gccgatcaac gtctcatttt cgccaaaagt tggcccaggg cttcccggta tcaacaggga 4920
   caccaggatt tatttattct gcgaagtgat cttccgtcac aggtatttat tcgaagacga 4980
   aagggcctcg tgatacgcct atttttatag gttaatgtca tgataataat ggtttcttag 5040
   acgtcaggtg gcacttttcg gggaaatgtg cgcgcccgcg ttcctgctgg cgctgggcct 5100
   gtttctggcg ctggacttcc cgctgttccg tcagcagctt ttcgcccacg gccttgatga 5160
   tcgcggcggc cttggcctgc atatcccgat tcaacggccc cagggcgtcc agaacgggct 5220
   tcaggcgctc ccgaaggt 5238
<210> 4
   <211> 5907
   <212> DNA
   <213> Artificial Sequence
<220>
<223> Plasmid
<220>
<221> misc-feature
<223> Plasmid
<400> 4
   atccggatat agttcctcct ttcagcaaaa aacccctcaa gacccgttta gaggccccaa    60
   ggggttatgc tagttattgc tcagcggtgg cagcagccaa ctcagcttcc tttcgggctt   120
   tgttagcagc cggatctcag tggtggtggt ggtggtgctc gagtcacgcg ccggccccca   180
   gcagccgcag cgcttcccgg ttgaaggccg gcaggtcgtc gggcttacgg ctggtgatca   240
   gctggtcctt gtcacgcacc acttcctggt cgtagaattc gccgcccgca ttcttcacgt   300
   cgtcctgcag gctgggccag gcggtcagct tgcggccgtt gatgaccccg ctgctgccca   360
   gcagccaccc gccgtggcag atcgcgaaga ccggcttgcc ggcggtcacg aattcacggg   420
   ccgcgggcag catccgattg tcgccacgca gcaggccgcc gggcagcagc agcgcgtcga   480
   attcgccttc ccggacttcg tcgaacgtac ggtccacctt gacggcttcc cccttgtccg   540
   cgtgacggaa ccccttgact tcgcccgcct gctggctgat cacgtcgact tcggcgccgg   600
   ccttacgcag cgcccgcagg ggttcgacca gttcgctgtc ttcgaattcg tccacggcca   660
   ggacggcgat cttcttgctc atgctgccgc cacgctgctg catatgtata tctccttctt   720
   aaagttaaac aaaattattt ctagagggga attgttatcc gctcacaatt cccctatagt   780
   gagtcgtatt aatttcgcgg gatcgagatc tcgatcctct acgccggacg catcgtggcc   840
   ggcatcaccg gcgccacagg tgcggttgct ggcgcctata tcgccgacat caccgatggg   900
   gaagatcggg ctcgccactt cgggctcatg agcgcttgtt tcggcgtggg tatggtggca   960
   ggccccgtgg ccgggggact gttgggcgcc atctccttgc atgcaccatt ccttgcggcg  1020
   gcggtgctca acggcctcaa cctactactg ggctgcttcc taatgcagga gtcgcataag  1080
   ggagagcgtc gagatcccgg acaccatcga atggcgcaaa acctttcgcg gtatggcatg  1140
   atagcgcccg gaagagagtc aattcagggt ggtgaatgtg aaaccagtaa cgttatacga  1200
   tgtcgcagag tatgccggtg tctcttatca gaccgtttcc cgcgtggtga accaggccag  1260
   ccacgtttct gcgaaaacgc gggaaaaagt ggaagcggcg atggcggagc tgaattacat  1320
   tcccaaccgc gtggcacaac aactggcggg caaacagtcg ttgctgattg gcgttgccac  1380
   ctccagtctg gccctgcacg cgccgtcgca aattgtcgcg gcgattaaat ctcgcgccga  1440
   tcaactgggt gccagcgtgg tggtgtcgat ggtagaacga agcggcgtcg aagcctgtaa  1500
   agcggcggtg cacaatcttc tcgcgcaacg cgtcagtggg ctgatcatta actatccgct  1560
   ggatgaccag gatgccattg ctgtggaagc tgcctgcact aatgttccgg cgttatttct 1620
   tgatgtctct gaccagacac ccatcaacag tattattttc tcccatgaag acggtacgcg 1680
   actgggcgtg gagcatctgg tcgcattggg tcaccagcaa atcgcgctgt tagcgggccc 1740
   attaagttct gtctcggcgc gtctgcgtct ggctggctgg cataaatatc tcactcgcaa 1800
   tcaaattcag ccgatagcgg aacgggaagg cgactggagt gccatgtccg gttttcaaca 1860
   aaccatgcaa atgctgaatg agggcatcgt tcccactgcg atgctggttg ccaacgatca 1920
   gatggcgctg ggcgcaatgc gcgccattac cgagtccggg ctgcgcgttg gtgcggatat 1980
   ctcggtagtg ggatacgacg ataccgaaga cagctcatgt tatatcccgc cgttaaccac 2040
   catcaaacag gattttcgcc tgctggggca aaccagcgtg gaccgcttgc tgcaactctc 2100
   tcagggccag gcggtgaagg gcaatcagct gttgcccgtc tcactggtga aaagaaaaac 2160
   caccctggcg cccaatacgc aaaccgcctc tccccgcgcg ttggccgatt cattaatgca 2220
   gctggcacga caggtttccc gactggaaag cgggcagtga gcgcaacgca attaatgtaa 2280
   gttagctcac tcattaggca ccgggatctc gaccgatgcc cttgagagcc ttcaacccag 2340
   tcagctcctt ccggtgggcg cggggcatga ctatcgtcgc cgcacttatg actgtcttct 2400
   ttatcatgca actcgtagga caggtgccgg cagcgctctg ggtcattttc ggcgaggacc 2460
   gctttcgctg gagcgcgacg atgatcggcc tgtcgcttgc ggtattcgga atcttgcacg 2520
   ccctcgctca agccttcgtc actggtcccg ccaccaaacg tttcggcgag aagcaggcca 2580
   ttatcgccgg catggcggcc ccacgggtgc gcatgatcgt gctcctgtcg ttgaggaccc 2640
   ggctaggctg gcggggttgc cttactggtt agcagaatga atcaccgata cgcgagcgaa 2700
   cgtgaagcga ctgctgctgc aaaacgtctg cgacctgagc aacaacatga atggtcttcg 2760
   gtttccgtgt ttcgtaaagt ctggaaacgc ggaagtcagc gccctgcacc attatgttcc 2820
   ggatctgcat cgcaggatgc tgctggctac cctgtggaac acctacatct gtattaacga 2880
   agcgctggca ttgaccctga gtgatttttc tctggtcccg ccgcatccat accgccagtt 2940
   gtttaccctc acaacgttcc agtaaccggg catgttcatc atcagtaacc cgtatcgtga 3000
   gcatcctctc tcgtttcatc ggtatcatta cccccatgaa cagaaatccc ccttacacgg 3060
   aggcatcagt gaccaaacag gaaaaaaccg cccttaacat ggcccgcttt atcagaagcc 3120
   agacattaac gcttctggag aaactcaacg agctggacgc ggatgaacag gcagacatct 3180
   gtgaatcgct tcacgaccac gctgatgagc tttaccgcag ctgcctcgcg cgtttcggtg 3240
   atgacggtga aaacctctga cacatgcagc tcccggagac ggtcacagct tgtctgtaag 3300
   cggatgccgg gagcagacaa gcccgtcagg gcgcgtcagc gggtgttggc gggtgtcggg 3360
   gcgcagccat gacccagtca cgtagcgata gcggagtgta tactggctta actatgcggc 3420
   atcagagcag attgtactga gagtgcacca tatatgcggt gtgaaatacc gcacagatgc 3480
   gtaaggagaa aataccgcat caggcgctct tccgcttcct cgctcactga ctcgctgcgc 3540
   tcggtcgttc ggctgcggcg agcggtatca gctcactcaa aggcggtaat acggttatcc 3600
   acagaatcag gggataacgc aggaaagaac atgtgagcaa aaggccagca aaaggccagg 3660
   aaccgtaaaa aggccgcgtt gctggcgttt ttccataggc tccgcccccc tgacgagcat 3720
   cacaaaaatc gacgctcaag tcagaggtgg cgaaacccga caggactata aagataccag 3780
   gcgtttcccc ctggaagctc cctcgtgcgc tctcctgttc cgaccctgcc gcttaccgga 3840
   tacctgtccg cctttctccc ttcgggaagc gtggcgcttt ctcatagctc acgctgtagg 3900
   tatctcagtt cggtgtaggt cgttcgctcc aagctgggct gtgtgcacga accccccgtt 3960
   cagcccgacc gctgcgcctt atccggtaac tatcgtcttg agtccaaccc ggtaagacac 4020
   gacttatcgc cactggcagc agccactggt aacaggatta gcagagcgag gtatgtaggc 4080
   ggtgctacag agttcttgaa gtggtggcct aactacggct acactagaag gacagtattt 4140
   ggtatctgcg ctctgctgaa gccagttacc ttcggaaaaa gagttggtag ctcttgatcc 4200
   ggcaaacaaa ccaccgctgg tagcggtggt ttttttgttt gcaagcagca gattacgcgc 4260
   agaaaaaaag gatctcaaga agatcctttg atcttttcta cggggtctga cgctcagtgg 4320
   aacgaaaact cacgttaagg gattttggtc atgagattat caaaaaggat cttcacctag 4380
   atccttttaa attaaaaatg aagttttaaa tcaatctaaa gtatatatga gtaaacttgg 4440
   tctgacagtt accaatgctt aatcagtgag gcacctatct cagcgatctg tctatttcgt 4500
   tcatccatag ttgcctgact ccccgtcgtg tagataacta cgatacggga gggcttacca 4560
   tctggcccca gtgctgcaat gataccgcga gacccacgct caccggctcc agatttatca 4620
   gcaataaacc agccagccgg aagggccgag cgcagaagtg gtcctgcaac tttatccgcc 4680
   tccatccagt ctattaattg ttgccgggaa gctagagtaa gtagttcgcc agttaatagt 4740
   ttgcgcaacg ttgttgccat tgctgcaggc atcgtggtgt cacgctcgtc gtttggtatg 4800
   gcttcattca gctccggttc ccaacgatca aggcgagtta catgatcccc catgttgtgc 4860
   aaaaaagcgg ttagctcctt cggtcctccg atcgttgtca gaagtaagtt ggccgcagtg 4920
   ttatcactca tggttatggc agcactgcat aattctctta ctgtcatgcc atccgtaaga 4980
   tgcttttctg tgactggtga gtactcaacc aagtcattct gagaatagtg tatgcggcga 5040
   ccgagttgct cttgcccggc gtcaatacgg gataataccg cgccacatag cagaacttta 5100
   aaagtgctca tcattggaaa acgttcttcg gggcgaaaac tctcaaggat cttaccgctg 5160
   ttgagatcca gttcgatgta acccactcgt gcacccaact gatcttcagc atcttttact 5220
   ttcaccagcg tttctgggtg agcaaaaaca ggaaggcaaa atgccgcaaa aaagggaata 5280
   agggcgacac ggaaatgttg aatactcata ctcttccttt ttcaatatta ttgaagcatt 5340
   tatcagggtt attgtctcat gagcggatac atatttgaat gtatttagaa aaataaacaa 5400
   ataggggttc cgcgcacatt tccccgaaaa gtgccacctg aaattgtaaa cgttaatatt 5460
   ttgttaaaat tcgcgttaaa tttttgttaa atcagctcat tttttaacca ataggccgaa   5520
   atcggcaaaa tcccttataa atcaaaagaa tagaccgaga tagggttgag tgttgttcca   5580
   gtttggaaca agagtccact attaaagaac gtggactcca acgtcaaagg gcgaaaaacc   5640
   gtctatcagg gcgatggccc actacgtgaa ccatcaccct aatcaagttt tttggggtcg   5700
   aggtgccgta aagcactaaa tcggaaccct aaagggagcc cccgatttag agcttgacgg   5760
   ggaaagccgg cgaacgtggc gagaaaggaa gggaagaaag cgaaaggagc gggcgctagg   5820
   gcgctggcaa gtgtagcggt cacgctgcgc gtaaccacca cacccgccgc gcttaatgcg   5880
   ccgctacagg gcgcgtccca ttcgcca                                      5907
<210> 5
   <211> 1077
   <212> DNA
   <213> Burkholderia glumae
<400> 5
   atggtcagat cgatgcgttc cagggtggcg gcgagggcgg tggcatgggc gttggcggtg     60
   atgccgctgg ccggcgcggc cgggttgacg atggccgcgt cgcccgcggc cgtcgcggcg    120
   gacacctacg cggcgacgcg ctatccggtg atcctcgtcc acggcctcgc gggcaccgac    180
   aagttcgcga acgtggtgga ctattggtac ggaatccaga gcgatctgca atcgcatggc    240
   gcgaaggtgt acgtcgcgaa tctctcggga ttccagagcg acgacgggcc gaacggccgc    300
   ggcgagcagc tgctcgccta cgtgaagcag gtgctcgcgg ccaccggcgc gaccaaggtg    360
   aacctgatcg gccacagcca gggcggcctg acctcgcgct acgtcgcggc cgtcgcgccg    420
   caactggtgg cctcggtgac gacgatcggc acgccgcatc gcggctccga gttcgccgac    480
   ttcgtgcagg acgtgctgaa gaccgatccg accgggctct cgtcgacggt gatcgccgcc    540
   ttcgtcaacg tgttcggcac gctcgtcagc agctcgcaca acaccgacca ggacgcgctc    600
   gcggcgctgc gcacgctcac caccgcgcag accgccacct acaaccggaa cttcccgagc    660
   gcgggcctgg gcgcgcccgg ttcgtgccag acgggcgccg cgaccgaaac cgtcggcggc    720
   agccagcacc tgctctattc gtggggcggc accgcgatcc agcccacctc caccgtgctc    780
   ggcgtgaccg gcgcgaccga caccagcacc ggcacgctcg acgtcgcgaa cgtgaccgac    840
   ccgtccacgc tcgcgctgct cgccaccggc gcggtgatga tcaatcgcgc ctcggggcag    900
   aacgacgggc tcgtctcgcg ctgcagctcg ctgttcgggc aggtgatcag caccagctac    960
   cactggaacc atctcgacga gatcaaccag ctgctcggcg tgcgcggcgc caacgcggaa   1020
   gatccggtcg cggtgatccg cacgcacgtg aaccggctca agctgcaggg cgtgtga      1077
<210> 6
   <211> 358
   <212> PRT
   <213> Burkholderia glumae
<400> 6
   Met Val Arg Ser Met Arg Ser Arg Val Ala Ala Arg Ala Val Ala Trp
   1               5                   10                  15
   Ala Leu Ala Val Met Pro Leu Ala Gly Ala Ala Gly Leu Thr Met Ala
               20                  25                  30
   Ala Ser Pro Ala Ala Val Ala Ala Asp Thr Tyr Ala Ala Thr Arg Tyr
           35                  40                  45
   Pro Val Ile Leu Val His Gly Leu Ala Gly Thr Asp Lys Phe Ala Asn
       50                  55                  60
   Val Val Asp Tyr Trp Tyr Gly Ile Gln Ser Asp Leu Gln Ser His Gly
   65                  70                  75                  80
   Ala Lys Val Tyr Val Ala Asn Leu Ser Gly Phe Gln Ser Asp Asp Gly
                   85                  90                  95
   Pro Asn Gly Arg Gly Glu Gln Leu Leu Ala Tyr Val Lys Gln Val Leu
               100                 105                 110
   Ala Ala Thr Gly Ala Thr Lys Val Asn Leu Ile Gly His Ser Gln Gly
           115                 120                 125
   Gly Leu Thr Ser Arg Tyr Val Ala Ala Val Ala Pro Gln Leu Val Ala
       130                 135                 140
   Ser Val Thr Thr Ile Gly Thr Pro His Arg Gly Ser Glu Phe Ala Asp
   145                 150                 155                 160
   Phe Val Gln Asp Val Leu Lys Thr Asp Pro Thr Gly Leu Ser Ser Thr
                   165                 170                 175
   Val Ile Ala Ala Phe Val Asn Val Phe Gly Thr Leu Val Ser Ser Ser
               180                 185                 190
   His Asn Thr Asp Gln Asp Ala Leu Ala Ala Leu Arg Thr Leu Thr Thr
           195                 200                 205
   Ala Gln Thr Ala Thr Tyr Asn Arg Asn Phe Pro Ser Ala Gly Leu Gly
           210                 215                 220
   Ala Pro Gly Ser Cys Gln Thr Gly Ala Ala Thr Glu Thr Val Gly Gly
   225                 230                 235                 240
   Ser Gln His Leu Leu Tyr Ser Trp Gly Gly Thr Ala Ile Gln Pro Thr
                   245                 250                 255
   Ser Thr Val Leu Gly Val Thr Gly Ala Thr Asp Thr Ser Thr Gly Thr
               260                 265                 270
   Leu Asp Val Ala Asn Val Thr Asp Pro Ser Thr Leu Ala Leu Leu Ala
           275                 280                 285
   Thr Gly Ala Val Met Ile Asn Arg Ala Ser Gly Gln Asn Asp Gly Leu
       290                 295                 300
   Val Ser Arg Cys Ser Ser Leu Phe Gly Gln Val Ile Ser Thr Ser Tyr
   305                 310                 315                 320
   His Trp Asn His Leu Asp Glu Ile Asn Gln Leu Leu Gly Val Arg Gly
                   325                 330                 335
   Ala Asn Ala Glu Asp Pro Val Ala Val Ile Arg Thr His Val Asn Arg
               340                 345                 350
   Leu Lys Leu Gln Gly Val
           355
<210> 7
   <211> 6
   <212> PRT
   <213> Burkholderia glumae
<400> 7
   Ala Ile Cys His Gly Pro
   1               5 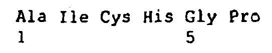
<210> 8
   <211> 29
   <212> DNA
   <213> Artificial Sequence
<220>
   <223> PCR Primer
<400> 8
   ctaggatcca attcgcctca tccaatgca 29
<210> 9
   <211> 23
   <212> DNA
   <213> Artificial Sequence
<220>
   <223> PCR Primer
<400> 9
   cgcaagcttt cacgcgccgg ccc 23
<210> 10
   <211> 21
   <212> DNA
   <213> Artificial Sequence
<220>
   <223> PCR Primer
<400> 10
   gtacatatgc agcagcgtgg c 21
<210> 11
   <211> 21
   <212> DNA
   <213> Artificial Sequence
<220>
   <223> PCR Primer
<400> 11
   atctcgagtc acgcgccggc c 21

## Patentansprüche

1. Protein, umfassend eine Aminosäuresequenz gemäß SEQ ID NO:2; oder eine davon abgeleitete Aminosäuresequenz mit wenigstens 80% Sequenzhomologie, wobei das Protein nach Expression in einem extrazelluläre Lipase produzierenden bakteriellen Wirt die extrazelluläre lipolytische Aktivität, bestimmt unter Standardbedingungen, erhöht.

2. Protein nach Anspruch 1, **gekennzeichnet durch** ein Molekulargewicht von etwa 20 - 22 kDa, bestimmt **durch** SDS-PAGE unter denaturierenden Bedingungen.

3. Protein nach Anspruch 1 oder 2, mit einem pl-Wert im Bereich von 5,4 bis 5,5 und/oder Proteaseaktivität.

4. Protein nach einem der vorhergehenden Ansprüche, erhältlich aus Bakterien der Gattung *Burkholderia.*

5. Protein nach Anspruch 4, erhältlich aus *Burkholderia glumae.*

6. Protein nach Anspruch 1, wobei der Wirt *B. glumae* ist.

7. Protein nach Anspruch 6, wobei die extrazelluläre lipolytische Aktivität um wenigstens etwa 5% im Vergleich zum Grundwert erhöht wird.

8. Protein nach einem der vorhergehenden Ansprüche, kodiert von einer Nukleinsäure, umfassend SEQ ID NO: 1 oder eine davon abgeleitete Sequenz mit wenigstens 80% Sequenzhomologie.

9. Nukleinsäuresequenz gemäß der Definition in Anspruch 8.

10. Expressionskonstrukt, umfassend unter der genetischen Kontrolle wenigstens einer regulativen Nukleinsäuresequenz eine ein Protein mit Proteaseaktivität kodierende Nukleinsäuresequenz gemäß Anspruch 9.

11. Rekombinanter Mikroorganismus, genetisch modifiziert mit wenigstens einem Expressionskonstrukt nach Anspruch 10.

12. Verfahren zur Herstellung eines Proteins mit Proteaseaktivität nach einem der Ansprüche 1 bis 8, wobei man einen rekombinanten Mikroorganismus unter das Protein exprimierenden Bedingungen kultiviert und das gebildete Protein isoliert.

13. Verfahren zur Herstellung einer Lipase (E.C. 3.1.1.3), wobei man einen zur Lipase-Produktion befähigten Wirt zur Expression eines funktionalen Proteins mit Proteaseaktivität nach einem der Ansprüche 1 bis 8 veranlasst und gleichzeitig oder zeitlich versetzt zur Lipase-Expression veranlasst und die gebildete Lipase isoliert.

14. Verfahren nach Anspruch 13, wobei der Wirt ein Bakterium der Gattung *Burkholderia* ist.

15. Verfahren nach einem der Ansprüche 13 und 14, wobei die Lipase eine Aminosäuresequenz gemäß SEQ ID NO: 6 oder eine davon abgeleitete Aminosäuresequenz mit wenigstens 80% Sequenzhomologie umfasst.

16. Verfahren nach einem der Ansprüche 13 bis 15, wobei die Lipase von einer Nukleinsäuresequenz umfassend eine Sequenz gemäß SEQ ID NO: 5 oder eine davon abgeleitete Nukleinsäuresequenz mit wenigstens 80% Sequenzhomologie kodiert wird.

## Claims

1. A protein, comprising an amino acid sequence according to SEQ ID NO: 2; or an amino acid sequence derived from it, with at least 80% sequence homology, the protein, after expression in an extracellular lipase-producing bacterial host, increasing the extracellular lipolytic activity, determined under standard conditions.

2. The protein according to claim 1, **characterized by** a molecular weight of about 20 - 22 kDa, determined by SDS-PAGE under denaturing conditions.

3. The protein according to claim 1 or 2, with a pI value in the range from 5.4 to 5.5 and/or protease activity.

4. The protein according to one of the preceding claims, obtainable from bacteria of the genus *Burkholderia.*

5. The protein according to claim 4, obtainable from *Burkholderia glumae.*

6. The protein according to claim 1, wherein the host is *B. glumae.*

7. The protein according to claim 6, wherein the extracellular lipolytic activity is increased by at least about 5% in comparison with the baseline value.

8. The protein according to one of the preceding claims, encoded by a nucleic acid, comprising SEQ ID NO: 1 or a sequence derived from it with at least 80% sequence homology.

9. The nucleic acid sequence as defined in claim 8.

10. An expression construct, comprising, under the genetic control of at least one regulatory nucleic acid sequence, a nucleic acid sequence according to claim 9, coding for a protein with protease activity.

11. A recombinant microorganism, genetically modified with at least one expression construct according to claim 10.

12. A method of production of a protein with protease activity according to one of the claims 1 to 8, wherein a recombinant microorganism is cultivated under conditions expressing the protein and the protein that forms is isolated.

13. A method of production of a lipase (E.C. 3.1.1.3), wherein a host that is capable of lipase-production is caused to express a functional protein with protease activity according to one of the claims 1 to 8 and is caused to express lipase, at the same time or at a different time, and the lipase that forms is isolated.

14. The method according to claim 13, wherein the host is a bacterium of the genus *Burkholderia.*

15. The method according to one of the claims 13 and 14, wherein the lipase comprises an amino acid sequence according to SEQ ID NO: 6 or an amino acid sequence derived from it with at least 80% sequence homology.

16. The method according to one of the claims 13 to 15, wherein the lipase is encoded by a nucleic acid sequence comprising a sequence according to SEQ ID NO: 5 or a nucleic acid sequence derived from it with at least 80% sequence homology.

## Revendications

1. Protéine comprenant une séquence d'aminoacides selon SEQ ID n° 2 ou une séquence d'aminoacides dérivée de celle-ci avec une homologie de séquence d'au moins 80 %, la protéine augmentant, après expression dans un hôte bactérien producteur de lipase extracellulaire, l'activité lipolytique extracellulaire, déterminée dans des conditions standard.

2. Protéine selon la revendication 1, **caractérisée par** une masse moléculaire d'environ 20-22 kDa, déterminée par SDS-PAGE dans des conditions dénaturantes.

3. Protéine selon la revendication 1 ou 2, ayant un pH dans l'intervalle de 5,4 à 5,5 et/ou une activité protéase.

4. Protéine selon l'une quelconque des revendications précédentes, pouvant être obtenue à partir de bactéries appartenant au genre *Burkholderia.*

5. Protéine selon la revendication 4, pouvant être obtenue à partir de *Burkholderia glumae.*

6. Protéine selon la revendication 1, l'hôte étant *B. glumae.*

7. Protéine selon la revendication 6, avec laquelle l'activité lipolytique extracellulaire est augmentée d'au moins environ 5 % par comparaison avec la valeur de base.

8. Protéine selon l'une quelconque des revendications précédentes, codée par un acide nucléique comprenant SEQ ID n° 1 ou une séquence dérivée de celle-ci avec une homologie de séquence d'au moins 80 %.

9. Séquence d'acide nucléique selon la définition dans la revendication 8.

10. Produit de construction d'expression, comprenant sous le contrôle génétique d'au moins une séquence d'acide nucléique régulatrice une séquence d'acide nucléique codant une protéine à activité protéase selon la revendication 9.

11. Micro-organisme recombinant, génétiquement modifié par au moins un produit de construction d'expression selon la revendication 10.

12. Procédé pour la production d'une protéine à activité protéase selon l'une quelconque des revendications 1 à 8, dans lequel on cultive dans des conditions permettant l'expression de la protéine un micro-organisme recombinant et on isole la protéine formée.

13. Procédé pour la production d'une lipase (E.C. 3.1.1.3), dans lequel on amène un hôte apte à la production de lipase à exprimer une protéine fonctionnelle à activité protéase selon l'une quelconque des revendications 1 à 8 et on l'amène simultanément ou ultérieurement à l'expression de lipase, et on isole la lipase formée.

14. Procédé selon la revendication 13, dans lequel l'hôte est une bactérie appartenant au genre *Burkholderia.*

15. Procédé selon les revendications 13 et 14, dans lequel la lipase comprend une séquence d'acide d'aminoacides selon SEQ ID n° 6 ou une séquence d'aminoacides dérivée de celle-ci avec une homologie de séquence d'au moins 80 %.

16. Procédé selon l'une quelconque des revendications 13 à 15, dans laquelle la lipase st codée par une séquence d'acide nucléique comprenant une séquence selon SEQ ID n° 5 ou une séquence d'acide nucléique dérivée de celle-ci avec une homologie de séquence d'au moins 80 %.
